# EUROPEAN PATENT APPLICATION

(11) **EP 4 684 830 A2**
(43) Date of publication of application: **28.01.2026**
(21) Application number: 25219557.3
(22) Date of filing: 27.03.2023
(51) Int. Cl.: A61P 1/00

(54) **SOLID EXTENDED-RELEASE COMPOSITION COMPRISING BRADYKININ B2-RECEPTOR ANTAGONISTS**

(30) Priority: 25.03.2022 EP 22164471
(62) Divisional of application: 23715488.5
(71) Applicant: Pharvaris GmbH, 6300 Zug (CH)
(72) Inventor: GIBSON, Christoph, 13189 Berlin (DE)
(74) Representative: Walter, Kai U.

(57) **Abstract**

The invention relates to pharmaceutical matrix tablets for oral administration comprising a bradykinin B2 receptor antagonist having a chemical structure according to Formula (1), or a salt or solvate thereof, wherein R is deuterium: 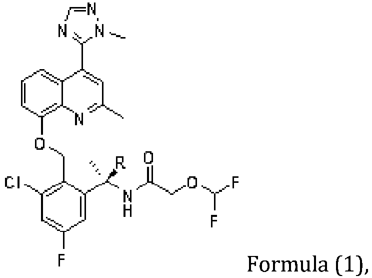 (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide; and at least one hydrophilic matrix-forming polymer. The matrix tablets reliably provide extended release of the bradykinin B2 receptor antagonist which makes them particularly suitable for prophylactic and/or chronic therapies. Therapeutic uses of the matrix tablets are provided.

## Description

### BACKGROUND OF THE INVENTION

The present invention relates to pharmaceutical compositions comprising a bradykinin (BK) B2 receptor antagonist having a chemical structure according to Formula (1), to methods of preparing such compositions, and to their uses as medicaments in the treatment of subjects that may benefit from a bradykinin B2 receptor antagonist.

Bradykinin (BK) is a peptide hormone that participates in inflammatory processes by activation of endothelial cells leading to vasodilation, increased vascular permeability, production of nitric oxide, and mobilization of arachidonic acid. BK also stimulates sensory nerve endings causing a burning dysaesthesia. Thus, the classical parameters of inflammation (e.g. redness, heat, swelling and pain) can all result from BK formation. BK is a short-lived component of the kallikrein-kinin system. The concentration of circulating BK is maintained at a low level under normal physiological conditions and may be rapidly increased under pathological situations by the enzymatic degradation of the circulating glycoprotein precursors called kininogens. The two most potent kininogen-metabolising enzymes are the trypsin-like serine proteases plasma kallikrein and tissue kallikrein. The precursors of these enzymes are normally present in all tissues and are ready to be activated by physiological or pathophysiological processes. The BK B2 receptor is constitutively expressed in most cell and tissue types and mediates most of the known effects of BK when this is produced in plasma or tissues. A large number of in vivo studies have shown that agents that blockade the BK B2 receptor provide therapeutic benefits in pathological conditions such as asthma, allergic rhinitis, pancreatitis, osteoarthritis, traumatic brain injury, Alzheimer's disease, and angioedema.

Numerous peptide and non-peptide antagonists of BK B2 receptor have been described in the prior art. Quinoline derivatives having activity as BK B2 receptor antagonists are, for example, disclosed in WO 2014/159637, WO 2010/031589, WO 2008/116620, WO 2006/40004, WO 03/103671, WO 03/87090, WO 00/23439, WO 00/50418, WO 99/64039, WO 97/41104, WO 97/28153, WO 97/07115, WO 96/13485, EP 0 795 547, EP 0 796 848, EP 0 867 432, and EP 1 213 289. However, these compounds showed a number of deficiencies hampering their utility as a drug, including low metabolic stability, low bioavailability, formation of glutathione adducts and bioactivation (toxicity) as disclosed in WO 2014/159637.

More recently, compounds of Formula (1) were proposed as novel, biologically active and well tolerated BK B2 receptor antagonists (see e.g. WO 2019/101906). While these compounds exhibit attractive pharmacological properties, they also show rather challenging physical or physicochemical properties, including very poor aqueous solubility in physiological media.

A compound according to Formula (1) is also known from A. Lesage et al., Frontiers in Pharmacology, vol. 11, 19 June 2020, doi: 10.3389/fphar.2020.00916. The publication describes the *in-vitro* pharmacological profile of the compound, but does not disclose any specific dosage form or formulation incorporating the compound.

Various dosage form designs and formulation techniques have been proposed for providing extended release of drug substances after oral administration, such as tablets or capsules having an extended-release coating, tablets or capsules comprising pellets with extended-release coatings, or matrix tablets. Extended-release coatings may be based, for example, on water-insoluble polymers capable of forming drug-permeable films on pellets, tablets or capsules, such as ethyl cellulose or certain methacrylate copolymers. Matrix-forming materials in extended-release formulations include hydrophobic excipients, such as lipids, waxes, or hydrophobic polymers; as well as hydrophilic, gel-forming polymers.

L.-A. Tziveleka et al., Carbohydrate Polymers, vol. 218, 29 April 2019, pages 355-370, doi: 10.1016/J.CARBPOL.2019.04.074 describes the use of ulvan, a sulphated polysaccharide found in green algae, in hydrogels for pharmaceutical product applications such as matrix tablets.

It remains challenging to develop suitable extended-release formulations for new drug candidates with difficult physical and pharmacokinetic properties, such as poor solubility, and still today many development programmes fail.

Thus, there is a need for formulation designs and pharmaceutical compositions that overcome the difficulties resulting from the challenging properties of compounds according to Formula (1) such as to enable effective oral delivery and achieve significant systemic exposure and bioavailability in human subjects. There is also a need for formulations or pharmaceutical compositions incorporating compounds of Formula (1) such as to achieve a reliable extent of oral absorption. A further need is the provision of formulations or pharmaceutical compositions incorporating compounds of Formula (1) that are stable in their performance and that can be manufactured by established pharmaceutical manufacturing technologies.

Compounds of Formula (1) have a very low water solubility. This makes it extremely challenging to develop an oral formulation that would result in a sufficient and reliable rate and extent of bioavailability and efficacious plasma levels. The pharmacodynamic profile of the BK B2 receptor antagonists of Formula (1) makes these compounds highly attractive for both acute and chronic treatment, and thus there is a need to overcome the challenges including poor aqueous solubility and develop formulations for these drugs that are viable both in the acute and in the chronic therapeutic setting.

An objective of the present invention is to address any one or more of these needs. A further objective is to overcome the deficiencies, gaps and limitations of the prior art with respect to the oral delivery of bradykinin B2 receptor antagonists, such as compounds having a chemical structure according to Formula (1). Further objectives will become apparent on the basis of the following description, the Examples and the patent claims.

### SUMMARY OF THE INVENTION

**In** one aspect, the invention relates to a pharmaceutical extended-release matrix tablet for oral administration comprising a bradykinin B2 receptor antagonist having a chemical structure according to Formula (1), or a salt or solvate thereof: 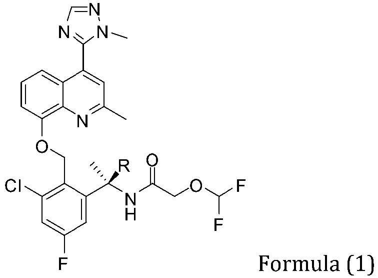 wherein R is deuterium or hydrogen; the matrix tablet is further characterised in that it comprises at least one hydrophilic matrix-forming polymer. As described in further detail below, the matrix tablet is preferably formulated such as to provide for the extended-release of the incorporated BK B2 receptor antagonist. The hydrophilic matrix-forming polymer functions to embed the active ingredient and effect its sustained release. For example, the matrix-forming polymer, when compressed into a tablet and upon contact with water or aqueous media, may be capable of swelling or gelling, and subsequently eroding slowly over time, such as to cause the extended-release of the BK B2 receptor antagonist. The hydrophilic matrix-forming polymer may, for example, be selected from hydroxypropyl cellulose (HPC), hydroxypropyl methylcellulose (HPMC), and combinations thereof. The matrix tablet may comprise further excipients, such as one or more tablet fillers, binders, glidants, and/or lubricants. Optionally, the matrix tablet may be coated, as described further below.

In some preferred embodiments, the BK B2 receptor antagonist of Formula (1), which has very poor solubility in physiological media, is incorporated in a physical form that is specifically adapted to facilitate the dissolution of the drug substance upon contact with water or aqueous media, such as in micronised or an already solubilised form. In some further preferred embodiments, the matrix tablet comprises a solid dispersion of the BK B2 receptor antagonist in amorphous form in a carrier, wherein the carrier comprises at least one pharmaceutically acceptable carrier polymer. The carrier polymer may, for example, be selected from HPMC, hydroxypropyl methylcellulose acetate succinate (HPMCAS), copovidone, and combinations thereof along with other optional excipients. The solid dispersion may be prepared by hot melt extrusion and incorporated into the matrix tablet in the form of a milled extrudate.

According to a further embodiment, the matrix tablet is an extended-release tablet that releases the BK B2 receptor antagonist over a period of 12 to 24 hours, as determined by an *in vitro* dissolution method described in further detail below.

In a further aspect, the present invention relates to certain therapeutic uses of the matrix tablet. In general, it may be used in the treatment of any diseases or conditions responsive to bradykinin B2 receptor modulation. For example, they are advantageous for the treatment of angioedema (AE), including hereditary angioedema (HAE), acquired angioedema (AAE), non-histaminergic idiopathic angioedema, allergic angioedema, drug-induced angioedema as well as angioedema of unidentified cause, and in particular in the chronic treatment of AE, especially of hereditary angioedema. The HAE can be of any type, including type I HAE, type II HAE, or type III HAE, preferably type I HAE or type II HAE.

### DESCRIPTION OF THE DRAWINGS

Figure 1 shows mean plasma concentration-time profiles of two different extended-release matrix tablets (formulations 5.1 and 5.2, here referred to as XR1 and XR2) comprising a solid dispersion formulation of a compound according to Formula (1), specifically (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide, here referred to as PHA-022121, compared to an immediate-release capsule formulation of the same drug, in healthy volunteers under fasting conditions.
Figure 2 shows mean plasma concentration-time profiles of an extended-release matrix tablet (formulation 5.1) comprising a solid dispersion formulation of a compound according to Formula (1) in healthy volunteers under fed and fasting conditions.
Figure 3 shows mean plasma concentration-time profiles of an extended-release matrix tablet (formulation 5.2) comprising a solid dispersion formulation of a compound according to Formula (1) in healthy volunteers under fed and fasting conditions.

### DETAILED DESCRIPTION OF THE INVENTION

In a first aspect, the present invention provides a pharmaceutical matrix tablet comprising a bradykinin (BK) B2 receptor antagonist having a chemical structure according to Formula (1), or a stereoisomer, salt or solvate thereof: 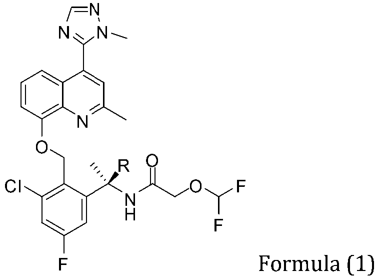 wherein R is deuterium or hydrogen; and at least one hydrophilic matrix-forming polymer.

The inventors have found that formulating such BK B2 receptor antagonist as a matrix tablet is particularly useful for providing reliable and complete drug release in spite of the unfavourable physicochemical properties of the compounds of Formula (1), which are, for example, very poorly soluble in aqueous media. Moreover, it was found that matrix tablets are highly compatible with these drug substances and capable of incorporating them in various forms, such as in the amorphous form, and lead to surprisingly stable and robust products, not only with respect to chemical stability, but also regarding a stable biopharmaceutical performance over long periods of storage under normal conditions. A further unexpected advantage is that matrix tablets incorporating a BK B2 receptor antagonist according to Formula (1) can be designed to provide for the extended-release of such active ingredient over long periods of time, such as over 12 hours or longer, and even over periods such as about 18 to 24 hours, as will be described in more detail below. Moreover, matrix tablets are easy to manufacture using commonly available pharmaceutical manufacturing equipment, and as such associated with moderate cost.

As commonly understood in the context of pharmaceutics, a pharmaceutical matrix tablet is a matrix tablet comprising an active pharmaceutical ingredient or a placebo version thereof, and formulated and processes such as to be acceptable as a medicinal product. In the present disclosure, therefore, the expressions "matrix tablet" and "pharmaceutical matrix tablet" are used interchangeably.

A matrix tablet, as generally understood in the field of pharmaceutics, describes a composition for oral administration in the form of a compressed tablet containing an active ingredient which is uniformly dispersed in one or more suitable excipients which, after compression, can form a matrix capable of controlling the release of the active ingredient. The matrix of a matrix tablet may be gellable. In the present disclosure, the expression "matrix" is used for such tablet both in its original dry form in which it is manufactured and provided, which may also be described as a porous matrix, as well as for the gelled matrix resulting from the contact of such matrix tablet with water.

Matrix tablets are often designed to provide for the extended-release of the incorporated active ingredient. In this case, such tablets may also be referred to as extended-release tablets, or extended-release matrix tablets, respectively. As used herein, extended-release is understood as the prolonged release of an active ingredient after oral administration, or in an appropriate *in-vitro* model, in particular characterised by gradual release over a period substantially longer than 30 minutes, in particular over several hours. In this context, extended-release, slow release, prolonged release and sustained release are used as interchangeable expressions. Extended-release is also a form of a modified release dosage form, also often referred to as controlled release preparations. In one of the generally preferred embodiments, the matrix tablet disclosed herein is an extended-release matrix tablet.

In a further embodiment, the matrix tablet is also designed as a single-unit dosage form, meaning that a single unit of the formulation comprises a single dose of the active ingredient. This is in contrast to a multiple-unit design, such as an extended-release capsule or tablet, incorporating a plurality of extended-release sub-units, e.g. pellets, which are dispersed upon contact with moisture prior to drug-release

The release of the active ingredient, also described as drug release, in the context of pharmaceutical products for oral administration, refers to the release of the active ingredient in dissolved form, i.e. dissolved in an aqueous dissolution medium such as gastric fluid, intestinal fluid, or surrogate versions thereof, such as diluted hydrochloric acid, buffers, simulated gastric fluid, simulated intestinal fluid and the like. For practical purposes, therefore, the release profile of an oral pharmaceutical composition may also be described as a dissolution profile and vice versa.

With respect to the active ingredient in the context of the present invention, i.e. the compound of Formula (1), R is selected from hydrogen and deuterium. In one preferred embodiment, or group of embodiments, R is deuterium, as shown in the following formula:

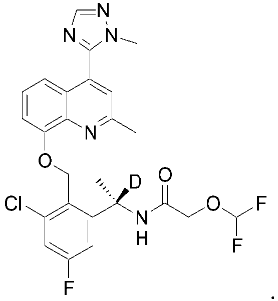

This compound which may also be referred to as (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide (CAS 2340111-58-0), or alternatively as acetamide, *N*-[(1*S*)-1-[3-chloro-5-fluoro-2-[[[2-methyl-4-(1-methyl-1*H*-1,2,4-triazol-5-yl)-8-quinolinyl]oxy]methyl]phenyl]ethyl-1-d]-2-(difluoromethoxy)-, is a particularly advantageous example of a compound according to Formula (1) in the context of the invention. It should be understood that this preference also applies in combination with all other optional features or preferences disclosed in this description below, whether specifically mentioned or not.

Alternatively, in Formula (1), R may be hydrogen, as depicted below: 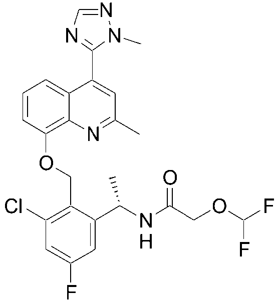 which compound may also be referred to as (*S*)-*N*-(1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy) acetamide.

In preferred embodiments, the BK B2 receptor antagonist is a compound according to Formula (1), a salt or solvate thereof, preferably a compound according to Formula (1) or a solvate thereof. That is, the compound of Formula (1) may be present in an essentially non-ionised form, or in ionised form, i.e. in the form of a salt. Moreover, it may optionally be in the form of a solvate. For example, the compound may be a hydrate, such as (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide monohydrate. In one of the generally preferred embodiments, the compound of Formula (1) is non-ionised (i.e. not in salt form) and is defined by R being deuterium.

In general, matrix tablets may be formulated on the basis of various types of matrix-forming excipients, such as hydrophilic, inert, lipidic, biodegradable and resin matrices. As mentioned, however, a key constituent of the matrix tablet according to an aspect of the invention, apart from the BK B2 receptor antagonist of Formula (1), is a hydrophilic matrix-forming excipient, more precisely, a hydrophilic matrix-forming polymer.

Such matrix tablet is different, for example, from a tablet whose extended-release functionality is predominantly effected by a sustained release membrane or coating. An extended-release matrix tablet may also comprise a coating, but if applied this coating has typically little effect on the drug release profile. For example, a so-called non-functional coating may be applied to a matrix tablet, such coating being primarily used for improving the appearance of the tablet, facilitating its handling and swallowing, covering the taste, or reducing friability.

In some preferred embodiments, the matrix tablet provided herein is an optionally coated single-layer tablet. A single-layer tablet consists of a single compressed tablet layer, which is different from bilayer or multilayer tablets. The manufacture of single-layer tablets is generally more cost-effective than that of tablets having two or more layers. Single-layer tablets may also be smaller and thus easier to swallow than tablets having one or more additional layers which are sometimes used to tailor the drug release profile. The inventors have found that compounds according to Formula (1) can very effectively be formulated as single-layer extended-release matrix tablets exhibiting prolonged drug release even over relatively long periods such as 12 to 24 hours. The optional coating is preferably a film-coating. For example, the coating may be a water-soluble, pH-independent film-coating based on a water-soluble film-forming polymer.

Formulation techniques for extended-release matrix tablets are generally known to those skilled in the art. Depending on the primary mechanism of drug release, "erodible" matrix tablets may be distinguished from "insoluble" matrix tablets that release their active ingredient mainly by diffusion out of a swollen but intact matrix. In practice, it is believed that often a combination of release mechanisms is present simultaneously, with some drug release occurring by diffusion while other portions of the drug are released as a consequence of tablet erosion. As used herein, erosion comprises any gradual loss of matrix material, regardless of the mechanism by which that loss occurs.

One of the primary functions of the hydrophilic matrix-forming polymer is to form a matrix capable of embedding the active ingredient and effecting its sustained release. Typically, such hydrophilic matrix-forming polymers are, when compressed into tablets and upon contact with water or aqueous media, capable of swelling or gelling, and subsequently eroding slowly over time, i.e. over at least several hours.

Various hydrophilic polymers may be used to create a compressed porous matrix capable of providing extended drug release. According to one embodiment, for example, one or more of the following polymeric excipients may be used as hydrophilic matrix-forming polymer: hydroxypropyl cellulose (HPC or hyprolose), hydroxypropyl methylcellulose (HPMC or hypromellose), hydroxypropyl methylcellulose acetate succinate (HPMCAS or hypromellose acetate succinate), alginic acid, bentonite, carbomer, carboxymethylcellulose calcium, carboxymethyl cellulose sodium, carrageenan, cellulose, copovidone, gelatin, guar gum, hydroxyethylcellulose, hydroxypropyl starch, methylcellulose, pectin, polyethylene oxide, polymethacrylates, potassium (or calcium, or sodium) alginate, povidone, tragacanth, or xanthan gum.

In one of the preferred embodiments, the hydrophilic matrix-forming polymer is selected from hydroxypropyl cellulose (HPC), hydroxypropyl methylcellulose (HPMC), and combinations thereof. In one of the preferred embodiments, the hydrophilic matrix-forming polymer in the matrix tablet is, or comprises, hydroxypropyl cellulose. In a related embodiment, the matrix tablet comprises a combination of two or more hydrophilic matrix-forming polymers, preferably including hydroxypropyl methylcellulose.

The inventors have found that these polymers and polymer combinations are among the particularly suitable matrix-forming excipients for the purpose of incorporating a BK B2 receptor antagonist of Formula (1).

Combinations of different grades of HPC and/or HPMC may also be used, in particular combinations of various grades of HPMC. For example, HPMC having a relatively low content of hydroxypropoxy groups (e.g. HPMC of type 2208, as classified by most pharmacopoeias; currently available, for example, as "K"-type Methocel^{™} grades), may be combined with a HPMC grade having a medium content of hydroxypropoxy groups and a relatively high content of methoxy groups (e.g. HPMC of type 2910; currently available, for example, as "E"-type Methocel^{™} grades); or HPMC having a relatively low viscosity (e.g. E4M, as classified by commercial providers of Methocel^{™}) with a HPMC having a relatively high viscosity (e.g. Methocel^{™} K100M), respectively. In this context, the viscosity of HPMC or HPC is understood as the typical viscosity of a 2% (w/v) aqueous solution of the polymer measured at 20 °C. Higher viscosity values are to some extent - but not exclusively - related to higher molecular masses of the respective polymer grades.

In some embodiments of the matrix tablet provided herein, the hydrophilic matrix-forming polymer comprises, or even consists of, a combination of a hydroxypropyl methylcellulose type 2208 and a hydroxypropyl methylcellulose type 2910. In some related embodiments, a high-viscosity grade of a hydroxypropyl methylcellulose type 2910 is used in combination with a hydroxypropyl methylcellulose type 2208.

In this context, it should be noted that there exist some highly soluble HPMC grades with very low apparent viscosities which may not be able to fully qualify as matrix-forming polymers. As used herein, matrix-forming HPMC polymers are preferably those HPMC grades with an apparent viscosity of at least about 100 cP, as determined according to the European Pharmacopoeia, using a 2% solution in water at 20 °C.

The hydrophilic matrix-forming polymer is typically provided in the form of a powder or granulate that is suitable for being incorporated in pharmaceutical tablets. The amount of the hydrophilic matrix-forming polymer in the matrix tablet, or, if a combination of hydrophilic matrix-forming polymers is used, the total amount of hydrophilic matrix-forming polymers in the matrix tablet provided according to an aspect of the invention may be selected, for example, in the range from about 10 wt.% to about 80 wt.%, relative to the weight of the matrix tablet. In some of the preferred embodiments, the amount is selected in the range from about 15 wt.% to about 60 wt.%, or from about 15 wt.% to about 50 wt.%, respectively.

In a further preferred embodiment, the hydrophilic matrix-forming polymer is HPMC, or a combination of two or more different grades of HPMC, and the total amount of HPMC in the matrix tablet is from about 15 wt.% to about 50 wt.%, and in particular in the range from about 20 wt.% to about 40 wt.%, relative to the weight of the matrix tablet, for example about 20 wt.%, 25 wt.%, 30 wt.%, 35 wt.% or 40 wt.%. The inventors have found that these ranges provide an excellent balance between drug loading, even if the active ingredient is incorporated in a special form, such as in solubilised form, for example in the form of a solid dispersion, as described in further detail below; and matrix functionality in terms of achieving extended-release profiles providing for drug release even over prolonged periods of about 12 to 24 hours.

In further embodiments, the matrix tablet further comprises one or more excipients to strengthen the mechanical stability of the compressed tablets by improving the binding behaviour of particles during compression.. These excipients, in contrast to the matrix-forming polymers, have little or no impact on the release-controlling properties of the matrix tablets. While some of these optional excipients may be chemically related to some of the preferred matrix-forming polymers, a skilled person would understand the major function and effect of an excipient present in a matrix tablet, and would know whether or not a specific excipient plays a significant role in controlling the release of the active ingredient incorporated in the matrix tablet. For example, some highly water-soluble HPMC grades with low molecular weight or viscosity, such as BonuCel^{®} D 5 H (apparent viscosity of a 2% aqueous solution at 20 °C is only about 4-6 cP), at least when used in low amounts such as 5 wt.%, relative to the weight of the uncoated matrix, are believed to act as binders rather than matrix-formers that contribute to the release-control effected by the matrix; they primarily increase the physical stability of the tablet. Similarly, low amounts of other excipients such as macrogols (also referred to as polyethylene glycols, e.g. macrogol 6000) or HPMCAS can be used without interfering with the extended drug release profile achieved by the hydrophilic matrix-forming excipient(s).

If present, such matrix-modifying excipient or combination of matrix-modifying excipients are preferably used at a relatively low level of, e.g., not more than about 20 wt.%, relative to the total weight of the matrix tablet (without coating), or not more than about 10 wt.%, such as about 2-8 wt.%, or about 5 wt.%, respectively.

According to another preferred embodiment, the matrix tablet further comprises a tablet filler. A tablet filler may be understood as a pharmaceutical excipient enhancing the general processability of powder or granule mixtures in view of tablet compression. The functions of a tablet filler may include those of diluents or bulking agents, compression aids, and dry binding agents (often simply referred to as binders). Tablet fillers are also referred to as tablet filler-binders. Such excipient may be a compound or a combination of compounds. Potentially suitable tablet fillers to be used in the matrix tablet include, without limitation, microcrystalline cellulose (MCC), starch such as corn starch, potato starch or pregelatinised starch; mannitol, lactose, sorbitol, cellulose, calcium sulphate, calcium phosphate such as dibasic calcium phosphate anhydrous or calcium hydrogen phosphate dihydrate; erythritol and calcium carbonate.

In one of the preferred embodiments, the tablet filler comprises microcrystalline cellulose and/or a calcium phosphate, such as calcium hydrogen phosphate dihydrate. In a related embodiment, the tablet filler comprises, or even consists of, a combination of microcrystalline cellulose and calcium hydrogen phosphate dihydrate. For example, the two excipients may be combined at a weight ratio of about 1 : 1. It is believed that such combination may be particularly useful in the matrix tablet in that MCC is insoluble in an aqueous environment but swells upon contact with water; calcium phosphate is inert and non-soluble; and the combination of both also contributes to maintaining the matrix structure over a prolonged period of time.

It has also been found that microcrystalline cellulose as a tablet filler, sometimes also referred to as filler-binder, alone or in combination with e.g. calcium hydrogen phosphate dihydrate, is particularly compatible with some of the preferred hydrophilic matrix-forming agents such as HPMC in that the microcrystalline cellulose does not interfere with the drug release-controlling functionality of the matrix-forming agent even if the latter is incorporated in the matrix tablet only in relatively small amounts, such as about 20 wt.% to 40 wt.%, based on the total weight of the matrix tablet. Hence, the matrix tablet may comprise about 20 wt.% to 40 wt.% HPMC and a combination of microcrystalline cellulose and calcium hydrogen phosphate dihydrate, according to another embodiment.

The microcrystalline cellulose, or a portion thereof, may also be incorporated in the form of silicified microcrystalline cellulose. If silicified microcrystalline cellulose, which also functions as a glidant, is used as tablet filler, it can at the same time replace other commonly used glidants such as colloidal silica, also referred to as colloidal silicon dioxide. Moreover, silicified microcrystalline cellulose may impart superior binding properties compared to non-silicified microcrystalline cellulose. If silicified microcrystalline cellulose is incorporated in the matrix tablet, its amount in the matrix tablet may, for example, range from about 15 wt.% to about 25 wt.%, based on the total weight of the matrix table, such as about 20 wt.% to 22 wt.%, based on the total weight of the matrix tablet. The amount of the tablet filler in the matrix tablet may, for example, range from about 20 wt.% to about 80 wt.%, based on the total weight of the matrix tablet. In one of the preferred embodiments, the amount of the tablet filler is in the range from about 25 wt.% to about 65 wt.%, or from about 30 wt.% to about 50 wt.%, respectively. These ranges also apply to the total amount of tablet fillers in the matrix tablet of more than one filler is used.

With respect to the combination of microcrystalline cellulose and calcium hydrogen phosphate dihydrate, the weight ratio of microcrystalline cellulose and calcium hydrogen phosphate dihydrate may, for example, be in the range from about 1 : 9 to 9 : 1, or from about 2 : 8 to about 8 : 2. Also preferred are weight ratios between about 4 : 6 to 6 : 4, such as about 5 : 5. For example, the matrix tablet may comprise about 20 wt.% to 40 wt.% HPMC and about 30 wt.% to 50 wt.% of a combination of microcrystalline cellulose and calcium hydrogen phosphate dihydrate, relative to the total weight of the matrix tablet, wherein the weight ratio between microcrystalline cellulose and calcium hydrogen phosphate dihydrate is in the range of 4 : 6 to 6 : 4.

According to further preferred embodiments, the matrix tablet further comprises a glidant and/or a lubricant. Also preferred is a related embodiment according to which the matrix tablet comprises a hydrophilic matrix-forming polymer, a tablet filler, a glidant and a lubricant.

A glidant is an excipient that improves the flow properties powders or granulates by decreasing interparticle friction and cohesion. Potentially suitable glidants include, without limitation, silicon dioxide, in particular colloidal silicon dioxide, anhydrous colloidal silica, peptized silica, talc, magnesium trisilicate, or powdered cellulose. In one embodiment, the glidant is colloidal silicon dioxide. The glidant may, for example, be present in the matrix tablet in an amount of about 0.1 wt.% to about 5 wt.%, in particular from about 0.1 wt.% to about 3 wt.%, relative to the total weight of the matrix tablet. If two or more glidants are present in the matrix tablet, these amounts refer to the total amount of the glidants. According to a further preferred embodiment, the matrix tablet comprises about 0.2 wt.% to 2 wt.% of colloidal silicon dioxide.

In the context of pharmaceutical tablet compression, a lubricant may be understood as an excipient which decreases the friction between the surface of a powder, granulate or tablet on the one hand and the wall of the die of a tablet press and the tablet punch on the other hand. Thus, a lubricant reduces wear and tear of tablet dies and punches. Potentially suitable lubricants include, without limitation, magnesium stearate, polyoxyethylene glycol, aluminum stearate, calcium stearate, calcium behenate, castor seed oil, talc, sodium benzoate, glyceryl mono fatty acid, glyceryl monostearate, glyceryl dibehenate, glyceryl palmito-stearic ester, hydrogenated cotton seed oil, sodium lauryl sulfate, sodium stearyl fumarate, fumaric acid, and stearic acid. The lubricant may, for example, be present in the matrix tablet in an amount of about 0.1 wt.% to about 5 wt.%, in particular from about 0.1 wt.% to about 4 wt.%, relative to the total weight of the matrix tablet. If two or more lubricants are present in the matrix tablet, these amounts refer to the total amount of the lubricants. In to a further preferred embodiment, the matrix tablet comprises about 0.2 wt.% to 2 wt.% of lubricant, in particular magnesium stearate.

The matrix tablet may comprise one or more further excipient, also referred to as inactive ingredients, such as one or more excipients selected from wetting agents such as surfactants, binding agents, granulating agents, anti-caking agents, flavours, sweetening agents, dyes, stabilisers, waxes, antioxidants, preservatives, and pH-adjusting agents.

As mentioned, the active ingredient, i.e. the BK B2 receptor antagonist according to Formula (1), may be incorporated in the matrix tablet as such, for example in the form of a powder which is provided and mixed with the inactive ingredients that are also typically provided in powder or granule form, such as to form a powder or granule mixture that can be compressed into a tablet. Alternatively, the active ingredient may be incorporated in a physical form which is specifically adapted to facilitate the dissolution of the drug substance upon contact with water or aqueous media, such as in micronised or solubilised form. Such formulation techniques are sometimes proposed for oral immediate release compositions of poorly soluble active ingredients, but as the inventors have found, they are also surprisingly advantageous for extended-release matrix tablets of the compounds of Formula (1) in that the design of the matrix tablet achieves the extended-release of the active ingredient in general, while the micronised or solubilised form ensures that the active ingredient is reliably and completely released, with only small or comparatively moderate impact of factors such as pH, food, and gastric motility.

Accordingly, in one of the preferred embodiments, the BK B2 receptor antagonist according to Formula (1) is incorporated in the matrix tablet in micronised form. Micronisation generally refers to the reduction of average particle diameters to the micrometre range, even through there are no precise, generally accepted upper and lower boundaries of such range. In the context of the present invention, a micronised form should be understood as the form of a powder having an average particle size (D50) in the range of about 0.1 µm to about 50 µm. In some further preferred embodiments, the BK B2 receptor antagonist is incorporated in the matrix tablet as a powder having an average particle size (D50) in the range of about 0.2 µm to about 40 µm, from about 0.5 µm to about 10 µm, respectively. Particularly preferred is a D50 value in the range from about 1 µm to about 10 µm, measured by laser diffraction.

In an also preferred embodiment, the particle size distribution is narrow, with only small fractions of particles that are substantially smaller or larger that the D50 value. In one embodiment, the ratio of the D90 value to the D10 value of the micronised BK B2 receptor antagonist is not higher than about 15, or not higher than about 10, respectively. It appears that such small ratio of the D90 value to the D10 value is favourable for a good distribution of the active agent within the matrix tablet and helps to achieve the desirable drug release characteristics described above. In further embodiments, D50 is in the range from about 3 µm to about 7 µm, D90 is in the range from about 6 µm to about 14 µm, and D10 is in the range from about 0.5 µm to about 2 µm, respectively. As known by those skilled in the art, the D10 and D90 values refer to the respective percentile values and specify the particles size below which 10% or 90% of all particles are found. If measured by laser diffraction, the percentages are typically volume-weighted percentages.

The micronised form of the BK B2 receptor antagonist of Formula (1) may be produced by generally known micronisation techniques. For example, various milling methods for producing micronised active ingredients are available and may be applied to the compounds of Formula (1). Optionally, co-milling of the BK B2 receptor antagonist with a hydrophilising agent and optionally one or more further excipients may be used. The milling or co-milling can be performed in conventional milling apparatuses, such as in a ball mill, cross beater mill, air jet mill, pin mill, classifier mill, disk mill, mortar grinder, rotor mill. Among the preferred mills to be used for micronising the BK B2 receptor antagonist are planetary mills and air jet mills.

In a preferred embodiment, a crystalline form of the BK B2 receptor antagonist of Formula (1) is used for preparing the micronised active ingredient. In particular, the crystalline monohydrate of (*S*)-*N*-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide may be incorporated within the matrix tablet.

For the avoidance of doubt, the incorporation of a micronised active ingredient with a specific particle size distribution within a compressed tablet may or may not allow the recovery of the active ingredient still having the same particle size distribution as at the time when it was incorporated. The process of tablet compression may change particle sizes and may also make particle size measurements difficult. In the context of the present invention, a matrix tablet incorporating a material having particular particle size characteristics should be interpreted such as to include a matrix tablet obtained from a process in which such material was provided, mixed with the remaining constituents and compressed into a matrix tablet, regardless of whether or not the specified particular particle size characteristics can still be observed in the matrix tablet.

In some alternative but also preferred embodiments, the BK B2 receptor antagonist is incorporated within the matrix tablet in solubilised form. For example, the active ingredient may be provided and incorporated in the form of formulated and pre-fabricated particles comprising the BK B2 receptor antagonist and one or more excipients.

As used herein, a solubilised form should be understood as a form from which the active ingredient is released into an aqueous medium at a substantially increased rate of dissolution, due to the specific physical form of the active ingredient itself, the presence of one or more solubilising excipients, or both. Solubilising techniques that may be used in pharmaceutical compositions for oral use are generally known to those skilled in the art, even though they are typically proposed for immediate release formulations rather than extended-release formulations as the latter essentially require slow drug release and not primarily the acceleration of drug dissolution. Examples of solubilising techniques and solubilised forms that may be used for practising these embodiments of the invention include, for example, and without limitation, cyclodextrin inclusion complexes, the use of acids such as tartaric acid, citric acid or fumaric acids to keep a basic active ingredient in the ionised and more soluble form, the use of bases such as meglumine to keep an acidic active ingredient in the ionised form; nano- or microparticles based on amphiphilic lipids, association of the drug with micelle-forming surfactant mixtures, self-emulsifying or self-microemulsifying systems, and solid dispersions.

In some of these systems, the drug substance is present in the amorphous rather than a crystalline form. While it is not at all straight-forward to use amorphous active ingredients due to processability and stability challenges, the amorphous form is capable of dissolving much more rapidly than a crystalline form of the same compound having a similar particle size. The inventors have found that the amorphous form of the BK B2 receptor antagonist may indeed be used advantageously in the matrix tablets. Accordingly, a further preferred embodiment provides that the matrix tablet comprises the BK B2 receptor antagonist predominantly in the amorphous form, preferably in amorphous form. The amorphous form means any non-crystalline form, such as a form in which the compound is molecularly dispersed or wherein the compound forms non-crystalline domains in a carrier. For the avoidance of doubt, the presence of the amorphous form does not exclude the presence of any crystallinity. For example, a particle may comprise the active ingredient in a partially amorphous form as well as in a partially crystalline form. In some preferred embodiments, the matrix tablet comprises the BK B2 receptor antagonist solely in the amorphous form.

According to some further preferred embodiments, the matrix tablet comprises a solid dispersion of the amorphous BK B2 receptor antagonist in a carrier, wherein the carrier comprises at least one pharmaceutically acceptable carrier polymer. It has been found by the inventors that solid dispersions of the BK B2 receptor antagonist of Formula (1) are not only effective in providing rapid and complete release of this pharmacologically active ingredient into physiological media, which is generally useful for designing immediate release compositions that incorporate such solid dispersions, but they are also capable of substantially improving the release of the compound of Formula (1) from extended-release formulations, which has been entirely unexpected. In particular, the solid dispersions may effect a substantially complete release of the active ingredient and reduce the variability of the release rate caused by external factors such as the pH and composition of the liquid medium into which the active compound is released from the extended-release composition, e.g. whether that liquid medium is water, gastric fluid, intestinal fluid, or surrogates thereof, such as simulated gastric fluid, fasted-state simulated intestinal fluid (FaSSIF) or fed-state simulated intestinal fluid (FeSSIF). In contrast, it has been found that conventional extended-release formulation techniques when applied to the compound of Formula (1) result in unreliable drug release, unsatisfactory release profiles, and unpredictable absorption rates.

In this context, a solid dispersion may be understood as a solid material comprising a compound such as a drug substance homogeneously dispersed in a preferably amorphous polymeric matrix. Typically, the dispersed compound is also in the amorphous state, i.e. non-crystalline. For example, the dispersed compound may be molecularly dispersed, or it may be dispersed in the form of non-crystalline domains.

In a generally preferred embodiment, the amorphous form of the BK B2 receptor antagonist is quantitatively dominant in the solid dispersion. In other words, even if some amount of crystalline BK B2 receptor antagonist is present in the solid dispersion, the quantity of the amorphous form is larger, or even substantially larger, than that of the crystalline form. In another generally preferred embodiment, at least about 90 %, or even at least 95 % or at least 99% of the BK B2 receptor antagonist comprised in the solid dispersion is amorphous. Also preferably, all or substantially all of the BK B2 receptor antagonist is in the amorphous form. In this context, the basis of the percentage is the total molar amount of the BK B2 receptor antagonist in the solid dispersion.

In a further embodiment, the state of the compound of Formula (1) in the solid dispersion is substantially or even entirely non-ionised. Moreover, the compound of Formula (1) as it is present in the solid dispersion would normally not represent a solvate. However, for the avoidance of doubt, the compound may be in the form of a solvate, when it is combined with the carrier polymer and any other optional constituents to form the solid dispersion as disclosed herein. For example, a hydrate of the compound, such as the monohydrate, optionally in crystalline form, may be used for preparing the composition. It is believed, however, that upon being incorporated into a solid dispersion, it no longer represents a crystalline material or a hydrate. Moreover, in one of the further preferred embodiments, the compound of Formula (1) is the sole drug substance contained in the solid dispersion. This is a generally applicable preference within the context of this disclosure.

As mentioned, the solid dispersion comprises a carrier comprising at least one pharmaceutically acceptable carrier polymer in which the BK B2 receptor antagonist is dispersed. In this context, the carrier typically means the excipient or excipient combination in which the active ingredient is dispersed. The carrier may consist of the polymer or a combination of polymers. In other cases, the carrier may comprise one or more further excipients, such as one or more stabilisers, plasticisers, non-polymeric surfactants and the like.

The carrier polymer may be selected from polymers that are suitable for oral administration and capable of incorporating the BK B2 receptor antagonist in the form of a solid dispersion. In this context, a polymer in which the active ingredient is embedded or dispersed such as to form a solid dispersion is sometimes herein referred to as a carrier polymer, which indicates its carrier function in the solid dispersion rather than its chemical nature. For the avoidance of doubt, a polymer such as HPMC which is potentially suitable for being used as a hydrophilic matrix-forming polymer in the matrix tablet may also be a useful candidate for being used as a carrier polymer for a solid dispersion.

The carrier polymer may, for example, be selected from the group consisting of hydrophilic neutral and anionisable polymers. In this context, hydrophilic means that the polymer exhibits at least some moderate solubility or swellability in an aqueous medium having a pH in the range of gastrointestinal pH values, i.e. between pH 1 and pH 8. Neutral means that an aqueous solution of the polymer has an approximately neutral effect, at least in the absence of other solutes. Preferably, the neutral polymer is also a non-ionic polymer. Anionisable means that the polymer comprises moieties that readily deprotonate when dissolved in aqueous media except at an acidic pH. Pharmaceutically acceptable hydrophilic polymers that are anionisable are sometimes also used as gastroresistant or enteric polymers. Moreover, the polymer is optionally a non-crosslinked polymer.

According to a further preferred embodiment, the pharmaceutically acceptable polymer in which the BK B2 receptor antagonist is amorphously and homogeneously dispersed is a thermoplastic polymer, i.e. a polymer that becomes pliable or mouldable at an elevated temperature and becomes solid again upon cooling down to e.g. room temperature. A thermoplastic polymer may also be understood as a polymer that goes through a glass transition temperature (Tg) when heat is applied while maintaining chemical stability. In one embodiment, the polymer is a water-soluble (optionally pH-dependant), neutral (e.g. non-ionic or even non-ionisable) or anionisable, and thermoplastic.

Specific carrier polymers that may be selected include hydroxypropyl cellulose (HPC), hydroxypropyl methylcellulose (HPMC), hydroxypropyl methylcellulose acetate succinate (HPMCAS), povidone (PVP), copovidone (PVP/PA), polyethylene glycol (PEG), and polyethylene oxide (PEO). Each of these polymers may individually used for forming the solid dispersion that incorporates the BK B2 receptor antagonist; alternatively, any combination of two or more of these polymers may be used, such as HPC in combination with HPMC, HPMC in combination with HPMCAS, PVP in combination with PVP/PA, or any other combination. In some preferred embodiments, the carrier polymer is selected from HPMC, HPMCAS, copovidone, and combinations thereof.

In one of the preferred embodiments, the pharmaceutically acceptable carrier polymer comprised in the solid dispersion is HPMCAS. HPMCAS, or hydroxypropyl methylcellulose acetate succinate, is an example of hydrophilic anionisable polymers that have a pH-dependent solubility in that it is insoluble in water at an acidic pH but soluble at a neutral or alkaline pH. HPMCAS may be understood as hydroxypropyl methylcellulose that has been esterified with a mixture of acetic acid and succinic acid. Generally, HPMCAS comprises from about 12% to about 28% of methoxy groups, from about 4% to about 23% of hydroxypropyl groups, from about 2% to about 16% acetyl groups, and about 4% to about 28% of succinoyl groups, calculated on a dry basis. HPMCAS is an exemplary pharmaceutically acceptable carrier polymer, and also a thermoplastic polymer, as well as being hydrophilic and anionisable. Various grades of HPMCAS are available including HPMCAS-LF, which is characterised by a mean particle size in the range of about 5 µm and a viscosity in the range of about 3 mPa*s.

The inventors have found that the BK B2 receptor antagonist according to Formula (1) is not only surprisingly compatible with HPMCAS in that it forms solid dispersions at various useful weight ratios between the drug substance and the polymer; it was also discovered that the respective solid dispersions are highly stable, both chemically and physically; i.e. showing little chemical degradation and only a very low tendency for the drug substance to form crystals over time. Also surprisingly, solid dispersions of the compound of Formula (1) with HPMCAS exhibit very useful drug release profiles in spite of the pH-dependant solubility of the polymer.

Alternatively, the solid dispersion may comprise copovidone (PVP/PA). It has been found that also this carrier polymer enables the formation of advantageous solid dispersions incorporating the compound of Formula (1). These solid dispersions are also chemically and physically stable. One of their additional advantages is that they enable a high drug loading with respect to the BK B2 receptor antagonist.

Copovidone, also referred to as copolyvidone, PVP/VA copolymer, or polyvinylpyrrolidone-vinyl acetate copolymer, is obtained by free-radical polymerization of vinylpyrrolidone and vinyl acetate in a molar ratio of about 6 : 4. Typical molecular weights are in the range of about 45,000 to 70,000. Examples of currently available commercial copovidone products include Kollidon VA 64 and Luviskol VA. Grades having different average particle sizes are also available.

In addition to the carrier polymers described above, one or more further polymers may be present in the solid dispersions. Moreover, a solid dispersion as claimed herein may also comprise a surfactant. In one embodiment, the surfactant is a non-ionic surfactant, in particular a non-ionic surfactant selected from the group of poloxamers and polysorbates. It is noted that these surfactants may also be considered as polymers; in other words, they may also function as contributing the effect of the polymeric drug carrier that forms the solid dispersion with the drug substance. They also function as surfactants in that they enhance the wetting of the solid dispersion upon contact with an aqueous medium such as water.

Poloxamers, also known as polyethylene-propylene glycol copolymers or polyoxyethylene-polyoxypropylene copolymers, are polyol block copolymers, each comprising a block of polyoxypropylene sandwiched between two blocks of polyoxyethylene. Various grades with different molecular weight ranges and different molecular ratios between the polyoxypropylene and polyoxyethylene blocks are available, such as poloxamer 124, 188, 237, 338 and 407.

The inventors have found that poloxamer 188 is particularly useful. It is a solid material with an average molecular weight in the range of about 7,680 to 9,510 in which the polyoxypropylene oxide block in average comprises about 27 repeating units and the polyoxyethylene blocks in average comprise about 80 repeating units. Particularly advantageous is poloxamer 188 in combination with copovidone.

Polysorbates, also referred to as polyoxyethylene sorbitan fatty acid esters, are another group of non-ionic surfactants which may be used as excipients in the formulation of the solid dispersions disclosed herein. Examples of potentially suitable polysorbates include polysorbate 20, 40, 60, 65, and 80, in particular polysorbate 20, which is also known as polyoxyethylene 20 sorbitan monolaurate and has an average molecular weight of 1,128, and polysorbate 80, whose chemical name is polyoxyethylene 20 sorbitan monooleate and which has a molecular weight of 1,310. A well-known branded product series is Tween.

Typically, the amount of the non-ionic surfactant in the solid dispersion, if present, is substantially lower than the amount of the carrier polymer, e.g. the hydrophilic neutral or anionisable polymer, which does not represent a surfactant. Also typically, the amount of the non-ionic surfactant in the solid dispersion is lower than the amount of the active ingredient. In one specific embodiment, the amount of the non-ionic surfactant in the solid dispersion is in the range from about 0.5 wt.% to about 10 wt.%, relative to the total weight of the solid dispersion, or from about 0.5 wt.% to about 5 wt.%. In a further specific embodiment, the weight ratio of the non-ionic surfactant to the polymer or, if a combination of two or more polymers is present, to the polymer combination, is in the range from about 1 : 100 to about 20 : 100, or from about 1 : 100 to about 10 : 100, respectively.

In one of the preferred embodiments, the solid dispersion exhibits a content of the BK B2 receptor antagonist of at least about 10 wt.%, based on the total weight of the solid dispersion. As mentioned above, it is also a preferred embodiment according to which the BK B2 receptor antagonist of Formula (1) is the sole drug substance in the solid dispersion. Hence, the range of at least about 10 wt.% would also represent a preferred drug loading of the solid dispersion.

According to further preferred embodiments, the solid dispersion exhibits a content of the BK B2 receptor antagonist of Formula (1) in the range from about 10 wt.% to about 50 wt.%, from about 15 wt.% to about 50 wt.%, or from about 15 wt.% to about 45 wt.%, based on the total weight of the solid dispersion. Again, in a related embodiment, the solid dispersion exhibits a drug content in the range from about 10 wt.% to about 50 wt.%, from about 15 wt.% to about 50 wt.%, or from about 15 wt.% to about 45 wt.%, wherein the BK B2 receptor antagonist of Formula (1) represents the sole drug substance present in the solid dispersion. It was unexpected that such high drug loadings can be achieved for the compound of Formula (1) when following the guidance provided in this disclosure, without significant chemical or physical instability.

In a further preferred embodiment, the solid dispersion comprises the BK B2 receptor antagonist of Formula (1) and from about 55 wt.% to about 85 wt.%, such as about 75 wt.% to 85 wt.% of the polymer HPMCAS, based on the total weight of the solid dispersion. A related embodiment provides a solid dispersion essentially consisting of about 15 wt.% to 45 wt.%, such as about 15 wt.% to 25 wt.% of the BK B2 receptor antagonist of Formula (1) and about 55 wt.% to about 85 wt.%, such as about 75 wt.% to 85 wt.% HPMCAS. For example, the solid dispersion may essentially consist of about 20 wt.% of the BK B2 receptor antagonist of Formula (1) and about 80 wt.% HPMCAS. It has been found by the inventors that the above ranges and the corresponding weight ratios between the drug substance and the carrier polymer HPMCAS yield solid dispersions that exhibit a sufficiently high drug load to allow an effective and convenient dosing, a high product stability and shelf life, as well as desirable release profiles.

According to an alternative preferred embodiment, the solid dispersion comprises about 30 wt.% to 50 wt.% of the BK B2 receptor antagonist of Formula (1), about 50 wt.% to 70 wt.% copovidone, and optionally up to about 5 wt.% poloxamer, such as poloxamer 188, based on the total weight of the solid dispersion. Again, in a related embodiment according to which no other constituents are present in the solid dispersion, a solid dispersion is provided which essentially consists of about 30 wt.% to 50 wt.% of the BK B2 receptor antagonist of Formula (1), about 50 wt.% to 70 wt.% copovidone, and optionally up to about 5 wt.% poloxamer, such as poloxamer 188. For example, the solid dispersion may essentially consist of about 40 wt.% of the BK B2 receptor antagonist of Formula (1), about 58.5 wt.% of copovidone, and about 1.5 wt.% of poloxamer 188. These embodiments are advantageous in that they allow for a particularly high drug load, while at the same time being chemically and physically stable and providing useful drug release properties.

As mentioned, the inventors have surprisingly discovered that the solid dispersions disclosed herein are particularly useful in formulations that release the BK B2 receptor antagonist over an extended period of time, such as the matrix tablets in some of their preferred embodiments. For facilitating the incorporation of the solid dispersion within the matrix tablet, according to some preferred embodiments, the solid dispersion is provided in the form of a powder or granules. Both powders and granules provide the solid dispersion as multiple particles, i.e. in a multiparticulate format. In this context, the expressions "powder" and "granules" are to be understood simply as referring to the approximate particle sizes, wherein a powder refers to a relatively fine multiparticulate material and granules refer to a relatively coarse material, regardless of the manufacturing method by which these materials were obtained. In other words, the expression "granules" should not be interpreted narrowly such as to require that a specific granulation method was used to produce the respective particles.

The solid dispersion for the matrix tablet may be prepared by various methods that are generally known to those skilled in the art. For example, the method for preparing the solid dispersion may comprise a step of hot melt extrusion of a powder or granule mixture comprising the BK B2 receptor antagonist and the carrier polymer, and optionally other excipients. Alternatively, the method may comprise a step of spray drying an organic solution of the BK B2 receptor antagonist and the carrier polymer and optionally other excipients, or spraying of an organic solution of the active ingredient and the polymer on particles of a pharmaceutically inactive material under conditions that cause the evaporation of the organic solvent(s). In this context, again, the BK B2 receptor antagonist is a compound according to Formular (1), in particular in a preferred form as disclosed above; and the carrier polymer is the at least one pharmaceutically acceptable carrier polymer in which the BK B2 receptor antagonist is homogeneously dispersed.

Hot melt extrusion is a technique that is generally known in the field of pharmaceutics. It is also generally known that the technique may in principle be used to produce melt extrudates. This includes the equipment that may potentially be used for hot-melt extrusion as well as some of the common process parameters such as temperature and the rotation speed of screws in case that screw extruders are used. Based on the technical guidance with respect to the composition of the solid dispersions provided herein, and taking into account the working examples described below, a skilled person will be able to prepare the melt-extruded solid dispersions comprising the active ingredient according to Formula (1) without difficulty.

Hot-melt extrusion may be used to convert a powder or granule mixture comprising the BK B2 receptor antagonist of Formula (1) and the at least one pharmaceutically acceptable carrier polymer into an extrudate which represents a solid dispersion in that the active ingredient becomes homogeneously and amorphously dispersed in the carrier polymer, and optionally other excipients. Hot-melt extrusion involves heating, softening or melting, mixing or homogenising, and extruding the mixture. It may also be described as a process whereby a blended composition is heated and/or compressed to a molten (or softened) state and subsequently forced through an orifice where the extruded product (extrudate) is formed into its final shape in which it solidifies upon cooling. The blended composition may be conveyed through one or more heating zones, which is typically achieved by a screw mechanism. The screw or set of screws may be rotated by a variable speed motor inside a cylindrical barrel where only a small gap exists between the outside diameter of the screw and the inside diameter of the barrel. In this conformation, high shear is created at the barrel wall and between the screw flights by which the components of the powder or granule blend are thoroughly mixed and disaggregated while being transported through the extruder. An extrudate refers to a composition formed by hot melt extrusion.

In one embodiment, the hot-melt extrusion step comprises the following sub-steps: (i) adding the optionally crystalline BK B2 receptor antagonist of Formula (1) (or e.g. a hydrate thereof) and the carrier polymer (and optionally other excipients) to a hot melt extrusion device; (ii) melting the BK B2 receptor antagonist and the polymer at temperatures close to or above the melting point of the BK B2 receptor antagonist and substantially less than its decomposition temperature; (iii) mixing the BK B2 receptor antagonist and the carrier polymer to form a homogenous blend; (iv) extruding the homogenous blend to form the solid dispersion shaped as an extrudate. Steps (ii) and (iii) may occur simultaneously, or step (iii) may begin as soon as the optionally crystalline BK B2 receptor antagonist of Formula (1) and the carrier polymer begin to melt. Again, the BK B2 receptor antagonist starting material in step (i) may be crystalline, and subsequently convert to the amorphous form during the hot melt extrusion process.

In one of the preferred embodiments, the method which comprises the step of hot melt extrusion is performed with a powder or granule mixture comprising about 15 wt.% to about 45 wt.%, or for example about 15 wt.% to 25 wt.% of the BK B2 receptor antagonist and about 55 wt.% to about 85 wt.% HPMCAS, or for example about 75 wt.% to about 85 wt.% HPMCAS, based on the total weight of the powder or granule mixture. In a related embodiment, a powder or granule mixture essentially consisting of about 15 wt.% to about 45 wt.%, or of about 15 wt.% to 25 wt.%, such as about 20 wt.%, of the BK B2 receptor antagonist and about 55 wt.% to about 85 wt.%, or about 75 wt.% to 85 wt.%, such as about 80 wt.% of HPMCAS is subjected to hot melt extrusion such as to obtain a melt extrudate that represents a solid dispersion.

In another one of the preferred embodiments, the step of hot melt extrusion is performed with a powder or granule mixture comprising about 30 wt.% to 50 wt.% of the BK B2 receptor antagonist, about 50 wt.% to 70 wt.% copovidone, and optionally up to 5 wt.% of poloxamer, in particular poloxamer 188, based on the total weight of the powder or granule mixture. In a related embodiment, powder or granule mixture consists of about 30 wt.% to 50 wt.% (such as about 40 wt.%) of the BK B2 receptor antagonist, about 50 wt.% to 70 wt.% (such as about 58.5 wt.%) copovidone, and optionally up to 5 wt.% (such as about 1-2 wt.%) of poloxamer, in particular poloxamer 188. For the avoidance of doubt, in these and the previous embodiments, the weight percentages are based on the weight of the powder or granule mixture that is subjected to the hot melt extrusion step.

The extrusion temperature, in some preferred embodiments, is selected to be above the glass transition temperature (T_{g}) of the respective carrier polymer or, if more than one carrier polymer is used, above the T_{g} of the carrier polymer having the highest T_{g}. In related preferred embodiments, the extrusion temperature is also selected above the melting temperature of the active ingredient. For example, the extrusion at a temperature of at least about 20 °C above the melting point of the API would facilitate solubilisation of the drug substance in the carrier polymer(s). In other preferred embodiments, the extrusion is carried out at a temperature of at least about 20 °C above the T_{g} of the polymer. In practice, extruders may have several temperature zones that may be set at different temperatures; in this context, the extrusion temperature should be understood as the temperature of the extruder zone with the highest temperature.

As mentioned, the solid dispersions as disclosed herein may also be prepared using a method that comprises a step of spray drying an organic solution of the BK B2 receptor antagonist and the carrier polymer (and optionally other excipients), i.e. the at least one pharmaceutically acceptable carrier polymer in which the BK B2 receptor antagonist according to Formula (1) is amorphously and homogeneously dispersed. Prior to the actual spray drying step, an organic solution of the BK B2 receptor antagonist according to Formula (1) and the carrier polymer (and any other constituents of the solid dispersion which may optionally be incorporated) in an organic solvent which is pharmaceutically suitable for the spray drying process and which is capable of fully dissolving both the active ingredient and the carrier polymer must be prepared, which can be done by any convention method of adding granular or powdery solids to an organic solvent under conditions (e.g. stirring, or elevated temperature) which lead to the dissolution of the solids.

Organic solvents that may be used in this context include, for example, methanol, ethanol, isopropanol, acetone, dichloromethane, chloroform, dimethyl formamide, dimethyl sulfoxide, ethyl acetate as well as any mixtures thereof. Optionally, the solvent or solvent mixture may contain limited amounts of water.

The spray drying step itself may be described as atomization under conditions that allow the evaporation of the organic solvent. For example, the atomization can be conducted by a nozzle or in a rotating disk. A further drying step may be used subsequently, such as fluidized bed drying, vacuum drying, tray drying, microwave drying, drum drying or double cone vacuum drying in order to reduce residual solvents to a pharmaceutically acceptable level. Generally, spray drying refers to making a highly dispersed liquid suspension or solution contact with air with enough volume, and causing evaporation and drying of droplets. Optionally, the organic solution of the BK B2 receptor antagonist according to Formula (1) and the carrier polymer (and any other excipients to be incorporated in the carrier or in the solid dispersion, if any) is injected into warm filtered air or inert gas flow to evaporate the solvent: The dried product particles may be conveyed to an accumulator, such as a cyclone particle collector.

Typically, the step of spray drying is performed at a temperature (i.e. inlet temperature) in the range of about 40°C to 150°C, or from about 50°C to 150°C, depending on the organic solvent that is used. In some embodiment, the inlet temperature is from about 80°C to 120°C. The outlet temperature for spray drying is typically lower, such as from about 40°C to 100°C, or from 40°C to 75°C. such as fluidized bed drying (such as from about room temperature to about 100°C), vacuum drying, (such as from about room temperature to about 200°C), such as from about room temperature to about 65°C, such as about 45°C.

In addition to methods relying on hot melt extrusion or spray drying, several other techniques or methods may also be used for the preparation of the solid dispersions incorporated within the matrix tablet as disclosed herein. These methods include, for example, cryogenic processing techniques, freeze drying, fluid-bed coating, microwave irradiation, co-precipitation, electrostatic spinning, supercritical anti-solvent precipitation, spray congealing, spray coating of carrier particles, melt agglomeration, and others.

As previously mentioned, the solid dispersions for the matrix tablets as disclosed herein may be provided in the form of a powder or granules. Both powders and granules provide the solid dispersion as multiple particles, i.e. in a multiparticulate format. Such powders or granules may be obtained directly from the technique that is applied for preparing the solid dispersions. For example, if spray drying or a method involving a spray drying step is used for preparing the solid dispersions, the immediate product obtained from such method is already in the form of a powder or granulate. Optionally, a subsequent step to adjust the particle size (distribution) of the product may be conducted, such as sieving, milling, or agglomeration. In case a method comprising a step of hot melt extrusion is used, the product directly obtained from the extrusion step is typically not a powder or granulate, but in the form of larger units or elongated pieces such as cylindrical rods which may subsequently be cut or pelletised into smaller units. A tool for cutting or pelletising the extrudate may be incorporated as a downstream module of the extrusion equipment. If further comminution is required in view of a desired particle size, an independent subsequent milling step may used, optionally followed by a classifying step, such as sieving.

In a further preferred embodiment, the solid dispersion is in the form of a powder or granules, and prepared by a method comprising a hot melt extrusion step as described above, and a milling step wherein the extrudate is converted into the powder or granulate, optionally with an intermediate step of cutting or pelletising the extrudate prior to milling. The milling may be conducted using any pharmaceutically suitable equipment, such as a hammer mill, centrifugal impact mill, jet mill, ball mill, cone mill, or oscillating and rotating sieve mill, to name only a few examples. Optionally, the milling occurs at controlled temperature or under cooling. The milling process may itself include screening or classifying the milled particles, depending on the milling method and the type of equipment used. Alternatively, a separate screening or classifying step may be performed after milling. The milling of the extrudate, optionally including a subsequent screening step, may be conducted such as to achieve an average particle size, for example, in the range of about 50 µm to 800 µm, or in a further preferred range of about 100 µm to 700 µm, respectively. In this context, the average particle size typically means the D50 value of the powder or granulate as measured by laser diffraction. Alternatively, if the particle size distribution is characterised by sieve analysis, the more relevant parameter is the mass median sieve diameter, which is also in the range of about 50 µm to 800 µm, or of about 100 µm to 700 µm, or of about 150 µm to 500 µm, according to some preferred embodiments.

In further preferred embodiments, the solid dispersion is incorporated into the matrix tablet in the form of particles having an average particle size D50 of not more than 250 µm. In this context, the respective average particle size D50 should be understood as characterising the particle size distribution of the solid dispersion particles prior to compression, i.e. prior to the formation of the matrix tablet. As a skilled person would understand, the average particle size D50 may be affected by compression, and after compression it may also be difficult to isolate the solid dispersion particles again in order to determine their particle size distribution.

In some further preferred embodiments, the average particle size D50 of the solid dispersion when incorporated into the matrix tablet is in the range of 125 to 250 µm, as determined by gravimetrical sieve analysis, or in the range of about 63 to 125 µm, respectively. Also useful are solid dispersion particles having an average particle size D50 of substantially below 100 µm, as preferably determined by laser diffraction, such as about 50±25 µm or even 10±5 µm, respectively.

In some preferred embodiments, the solid dispersion as incorporated in the matrix tablet comprises at least about 10 wt.% of BK B2 receptor antagonist, relative to the total weight of the solid dispersion. Other preferred amounts of the BK B2 receptor antagonist, in particular (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, are in the range from about 10 wt.% to about 50 wt.%, from about 15 wt.% to about 45 wt.%, or from about 20 wt.% to about 40 wt.%, respectively, relative to the total weight of the solid dispersion. Further preferred is the incorporation of solid dispersion particles comprising from about 15 wt.% to about 25 wt.% of BK B2 receptor antagonist, such as about 20 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide amorphously dispersed in 80 wt.% hypromellose acetate succinate. In further preferred embodiments, solid dispersion particles are incorporated in the matrix tablet which comprise about 25 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide amorphously dispersed in 75 wt.% hypromellose acetate succinate; or about 30 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide amorphously dispersed in 70 wt.% hypromellose acetate succinate; or about 35 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide amorphously dispersed in 65 wt.% hypromellose acetate succinate, respectively.

As mentioned, the inventors have found that the incorporation of the solid dispersion within matrix tablets, in particular extended-release matrix tablets is unexpectedly advantageous, in spite of the fact that the solid dispersion itself has drug solubilising properties which would not be expected to be useful for providing slow drug release over an extended period of time. However, it was found that the solid dispersion is remarkably compatible with the formulation techniques for extended-release matrix tablets disclosed hereinabove in that slow drug release may be achieved even over periods of, e.g., about 8 to 24 hours, and that the solid dispersion effects a more reliable release profile. Moreover, it was found that the use of the solid dispersion in extended-release formulations is associated with a low or moderate food effect, contrary to what would normally have been expected for extended-release compositions of the BK B2 receptor antagonist of Formula (1), in view of its solubility and intrinsic release behaviour. In one preferred embodiment, the matrix tablet comprises about 10 wt.% to about 60 wt.% of the solid dispersion, based on the total weight of the matrix tablet. In further preferred embodiments, the content of the solid dispersion in the matrix tablet is in the range of about 15 wt.% to about 50 wt.%, in particular in the range from about 15 wt.% to about 40 wt.%. In further preferred embodiments, the content of the solid dispersion in the matrix tablet is in the range from about 20 wt.% to about 40 wt.%, such as about 20 wt.%, about 25 wt.%, about 30 wt.%, about 35 wt.%, or about 40 wt.%, respectively. Again, the basis of the percentages is the total weight of the matrix tablet, without the optional coating.

In some further preferred embodiments, the matrix tablet is formulated to comprise the solid dispersion at an amount that is somewhat at the same approximate level as the amount of the hydrophilic matrix-forming polymer. For example, the weight ratio of the solid dispersion to the hydrophilic matrix-forming polymer may be in the range from 2 : 1 to 1 : 2. If more than one hydrophilic matrix-forming polymer is used, the preferred range of 2 : 1 to 1 : 2 applies to the weight ratio of the solid dispersion to the total weight of hydrophilic matrix-forming polymers in the matrix tablet.

As mentioned, the matrix tablet may incorporate the solid dispersion in the form of a powder or granules, such as in the form of a milled extrudate as described above. In this context, In addition to the solid dispersion, the matrix tablet preferably comprises further excipients, such as may be necessary or useful for tablet compression, but and also for the purpose of forming a matrix in which the solid dispersion powder or granules are embedded. The incorporation of powders or granules into compressed tablets may change certain characteristics of the solid dispersion as described above. For example, once a powder or a granulate has been incorporated within a compressed tablet, the flowability that typically characterises a powder or granules may not be present any longer. Moreover, even if particles representing the solid dispersion may, with some technical difficulty, be extracted from the matrix tablet, the particle size-related parameters may no longer be the same as before the solid dispersion was incorporated in the tablet. In this respect, the embodiments relating to matrix tablets comprising the solid dispersion with the optional or preferred features described in the context of the solid dispersion should be understood as also covering matrix tablets that are obtainable from powder or granule mixtures incorporating such solid dispersions by compressing these mixtures.

In one of the preferred embodiments, the BK B2 receptor antagonist of Formula (1) is the only active ingredient in the matrix tablet. This should also be understood as a general preference in the context of the present invention. Alternatively, the pharmaceutical composition may comprise one or more further active ingredients. The further or supplemental active ingredient or active pharmaceutical ingredient is preferably an active agent or active pharmaceutical ingredient which has utility in the prevention or treatment of one or more condition(s) responsive to BK B2 receptor modulation, including a condition selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema. For instance, at least one compound or pharmaceutically acceptable salt of the invention may advantageously be contained in a combination preparation that includes an antibiotic, anti-fungal, or anti-viral agent, an anti histamine, a non-steroidal anti-inflammatory drug, a disease modifying anti-rheumatic drug, a cytostatic drug, a drug with smooth muscle activity modulatory activity, an antibody, or mixtures of the aforementioned as further or supplemental active agent or active pharmaceutical ingredient.

The matrix tablet may further be described in terms of the dose strength or amount of the BK B2 receptor antagonist of Formula (1) that is incorporated per dose unit, i.e. per capsule or tablet. In some embodiments, the dose strength is is the range from about 1 mg to about 100 mg, or from about 5 mg to about 100 mg, respectively. In further preferred embodiments, the dose strength is is the range from about 10 mg to about 80 mg, such as about 10 mg, 20 mg, 30 mg, 40 mg, 50 mg, 60 mg, 70 mg, or 80 mg, respectively. For paediatric use, also lower doses such as from about 0.5 mg to about 5 mg may be useful.

In some preferred embodiments, the matrix tablet comprises:
- about 15 wt.% to 40 wt.% of the solid dispersion,
- about 20 wt.% to 40 wt.% of the hydrophilic matrix-forming polymer(s), and
- about 30 wt.% to 50 wt.% of the filler(s).

According to some further preferred embodiments, the matrix tablet comprises:
- about 20 wt.% to 40 wt.% of the solid dispersion;
- about 20 wt.% to 40 wt.% of hydroxypropyl methylcellulose, or of a combination of different grades of hydroxypropyl methylcellulose;
- about 30 wt.% to 50 wt.% of microcrystalline cellulose, calcium hydrogen phosphate dihydrate, or a combination thereof;
- up to about 2 wt.% of a glidant, in particular colloidal silicon dioxide, and
- up to about 2 wt.% of a lubricant, in particular magnesium stearate.

For the avoidance of doubt, and in this context, all weight percent values are based on the total weight (100%) of the matrix tablet without its optional coating. In other words, the total wt.% of all components contained in a matrix tablet disclosed herein does not exceed 100%.

Also in these embodiments, the solid dispersion may represent a solid dispersion according to some of the preferences described above, and for example essentially consist of about 15 wt.% to about 45 wt.%, or of about 15 wt.% to 25 wt.% of the BK B2 receptor antagonist of Formula (1) and about 55 wt.% to about 85 wt.%, or about 75 wt.% to 85 wt.% HPMCAS; or it may essentially consist of about 30 wt.% to 50 wt.% of the BK B2 receptor antagonist of Formula (1), about 50 wt.% to 70 wt.% copovidone, and optionally up to about 5 wt.% poloxamer, such as poloxamer 188.

As already mentioned, the matrix tablet may further comprise a coating, for example for the purpose of improving the appearance or the swallowability of the tablet. The coating, if present, is preferably a film-coating, such as a coating comprising e.g. water-soluble film-forming polymer and a plasticiser. The film-coating may also comprise more than one polymer and/or more than one plasticiser (e.g. glycerol). Moreover, the film-coating may comprise further constituents as commonly used in tablet coating that are not intended to modify the release of the drug, for example one or more dyes, pigments (such as iron oxides), flavours, sweetening agents, diluents (such as MCC) and opacifiers (such as calcium carbonate or calcium phosphate).

In some embodiments, the amount of coating applied to the matrix tablet is not more than about 10 wt.%. In this context, the percentage is based on the weight of the uncoated tablet, and used for the weight of the film coating in the dry state, i.e. after solvent removal. In further embodiments, the amount of the coating is 2 wt.% to 8 wt.% relative to the weight of the uncoated tablet.

In a further aspect, the invention provides a method for preparing the matrix tablet, in particular the extended-release matrix tablet comprising the solid dispersions as disclosed above; the method comprises the steps of: (a) providing a solid dispersion in powder or granular form, the solid dispersion comprising BK B2 receptor antagonist and a carrier comprising at least one pharmaceutically acceptable carrier polymer, wherein the BK B2 receptor antagonist is amorphous and dispersed in the carrier; (b) combining the solid dispersion with a hydrophilic matrix-forming polymer (or combination of hydrophilic matrix-forming polymers), a filler and optionally further excipients such as to form a compressible powder or granule mixture, and (c) compressing the powder or granule mixture to obtain the matrix tablet. Step (a) may comprise one or more sub-steps directed to the preparation of the solid dispersion, e.g. by hot melt extrusion, optionally followed by milling, or by spray drying, as described above.

For example, and according to one of the preferred embodiments, the method comprises the steps of: (a) providing a solid dispersion in powder or granular form, the solid dispersion being a milled extrudate comprising (1) about 15 wt.% to about 45 wt.%, such as about 15 wt.% to 25 wt.% of the BK B2 receptor antagonist of Formula (1) and a carrier comprising or essentially consisting of about 55 wt.% to about 85 wt.%, such as about 75 wt.% to about 85 wt.% of HPMCAS, or (ii) about 30 wt.% to 50 wt.% of the BK B2 receptor antagonist of Formula (1) and a carrier comprising or essentially consisting of about 50 wt.% to 70 wt.% copovidone, and optionally up to about 5 wt.% poloxamer, such as poloxamer 188, wherein the BK B2 receptor antagonist is amorphous and dispersed in the carrier; (b) combining the solid dispersion with about 20 wt.% to 40 wt.% of a hydrophilic matrix-forming polymer which is hypromellose, about 30 wt.% to 50 wt.% of a tablet a filler which is microcrystalline cellulose, calcium hydrogen phosphate dihydrate, or a combination thereof and optionally further excipients such as to form a compressible powder or granule mixture, and (c) compressing the powder or granule mixture to obtain the matrix tablet.

As also illustrated by the Examples, the matrix tablets provided herein can be prepared without any agglomeration step for improving the flowability or compressibility of the powder or granule mixture from which the tablets are compressed. In some related embodiments, therefore, step (c) of compressing the powder or granule mixture to obtain the matrix tablet is conducted without any prior dry or wet agglomeration step. In this particular context, agglomeration refers to any powder granulation process in which small primary powder particles are agglomerated to form larger secondary particles. Other preferred embodiments of the preparation method will be understood by those skilled in the art on the basis of the guidance provided in this disclosure regarding the selection of some preferred versions of, e.g., the BK B2 receptor antagonist, the excipients in the solid dispersion and their amounts, the preparation methods relating to the solid dispersion, and the excipients in the matrix tablet and their amounts.

As already mentioned, the inventors have found that the matrix tablet, in particular the extended-release matrix tablet according to any of the preferred embodiments as disclosed above, allows the tailoring of clinically advantageous extended-release profiles of the BK B2 receptor antagonist of Formula (1) with, for example, good robustness and reliability, and with a reduced food effect, compared to conventional formulation techniques. This advantage is particularly pronounced for extended-release matrix tablets comprising solid dispersions using hydroxypropyl methylcellulose acetate succinate (HPMCAS) as the carrier polymer of the solid dispersion, i.e. as the pharmaceutically acceptable carrier polymer in which the active ingredient is amorphous and homogeneously dispersed. Without wishing to be bound by theory, these release properties may relate to the fact that HPMCAS is a polymer that is insoluble in acidic media (e.g. gastric fluid) such as to cause a relatively slow drug release in such acidic environment, whereas the compound of Formula (1) itself is poorly soluble in particular in relatively neutral media (e.g. intestinal fluid) such as to cause a relatively slow drug release in such environment, so that the various pH-dependent effects on the drug release rate may be partially compensated. In other words, the design of a extended-release matrix tablet comprising the solid dispersion incorporating the solubilised, amorphous drug ensures effective drug release and oral bioavailability when the BK B2 receptor antagonist of Formula (1) is formulated as an extended-release dosage form.

Extended-release profiles, while also aiming at describing the behaviour of a composition or formulation in the relevant biological environment, i.e. in the clinical situation after administration of the composition or formulation to a subject, are typically measured in well-defined *in vitro* models. As used herein, an extended-release profile means the dissolution profile of an extended-release composition. A description of some widely accepted dissolution models for determining dissolution profiles is found, for example, in the United States Pharmacopeia (e.g. USP), general chapter <711> ("DISSOLUTION"). For characterising the dissolution profiles of the matrix tablets disclosed herein, the Apparatus II (paddle apparatus) described in the USP may, for example, be used. Furthermore, it is preferred to use, as the dissolution medium, 1,000 mL of USP phosphate buffer pH 6.8 with 0.3% (w/v) sodium dodecyl sulphate. In the case that the extended-release composition floats on the dissolution medium (e.g. as some extended-release matrix tablets tend to do), sinkers such as Japanese sinkers should be used for ensuring that the composition remains submerged during dissolution testing. Optionally, alternative testing conditions may be used if they are appropriate in view of the guidance on dissolution testing issued by the prominent regulatory offices, such as the US FDA.

In some preferred embodiments, the matrix tablet, in particular the extended-release matrix tablet comprising the solid dispersions as disclosed above, whether coated or not, exhibits a release of the BK B2 receptor antagonist of Formula (1) over a period of at least about 6 hours, or over at least about 8 hours, such as over about 8 to 24 hours. In a further preferred embodiment, the extended-release compositions release the BK B2 receptor antagonist over a period of 12 to 24 hours, as determined by dissolution testing using the preferred method and conditions as described hereinabove. In this context, the period of 12 to 24 hours over which the active ingredient is released means that the point in time when 95% of the active ingredient incorporated in the composition has been released falls within the specified time period. For example, the extended-release composition may be characterised by a dissolution profile which reaches 95% drug dissolution after about 12 hours, or after about 13, 14, 15, 16, 17, 18, 19, 20, 21, 22, 23 or 24 hours, respectively.

The extended-release profiles, or dissolution profiles, may also be described in terms of the duration of time until about 50% of the incorporated drug released, i.e. dissolved. In some of the preferred embodiments, the extended-release compositions reach 50% dissolution within a period of about 3 to 10 hours, or within a period of about 4 to 8 hours, such as after about 4, 5, 6, 7 or 8 hours, respectively.

In some of the preferred embodiments, the matrix tablet, in particular the extended-release matrix tablet comprising the solid dispersions as disclosed above, has a dose strength in the range from about 10 mg to 80 mg and release the BK B2 receptor antagonist of Formula (1) over a period of about 12 to 24 hours, as previously defined. Also preferred are embodiments according to which the matrix tablet comprises a solid dispersion, based on, or incorporating, HPMCAS, wherein the tablet has a dose strength of about 20 mg to 40 mg and exhibit a release over a period of about 12 to 24 hours.

According to a further aspect of the invention, the matrix tablet as disclosed herein, in particular the extended-release matrix tablet comprising the solid dispersion, may be used in the treatment of a subject suffering from any disease or condition responsive to bradykinin B2 receptor modulation. Such condition, including a condition selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; and edema. Expressed in other words, one aspect of the invention relates to a method of treating a subject suffering from any disease or condition responsive to bradykinin B2 receptor modulation, wherein the method comprises the administration of the matrix tablet as described above. Similarly, the invention provides the use of the matrix tablet in the manufacture of a medicament for the treatment of any disease or condition responsive to bradykinin B2 receptor modulation.

As used herein, the expressions "treatment", "treating" and the like should be interpreted to include any type of prophylactic or therapeutic treatment. As such, it encompasses the prevention, management or therapy of a disease or its reoccurrence, or of any symptoms associated with such disease or condition. Treatment includes acute and chronic treatment.

Chronic treatment, in the context of the invention, refers to continuous treatment of a disease or condition, comprising repeated administration of a medication according to a regular dosing regimen over an extended period of time, such as over at least one month, or over at least three months. In certain embodiments wherein the condition of the patient or subject does not improve, the compound of Formula (1) is administered chronically, that is, for an extended period of time, including throughout the duration of the life of the patient or subject in order to ameliorate or otherwise control or limit symptoms associated with a disease or condition in the patient or subject. For example, chronic treatment may include the administration of one, two or three dose units per day over such period. In one of the preferred embodiments, the matrix tablet as disclosed herein, in particular the extended-release matrix tablet comprising the solid dispersion, is used for a chronic treatment or therapy of the disease or condition responsive to BK B2 receptor modulation, e.g. AE, over an extended period of time, such as over a period of at least one month. In certain preferred embodiments, the treatment disclosed herein, in particular the treatment of angioedema, especially hereditary angioedema, is prophylactic treatment, *i.e.,* before the onset of symptoms, in order to prevent, delay or reduce the severity of symptoms.

As previously mentioned, and according to one of the preferred embodiments, a matrix tablet, in particular the extended-release matrix tablet according to any of the preferred embodiments as disclosed above, such as the extended-release matrix tablet comprising the solid dispersion of the BK B2 receptor antagonist of Formula (1) is provided, of which the inventors have found that it provides advantageous extended-release properties. It is believed that these properties also render such extended-release compositions particularly suitable for chronic, or long-term treatment. One of the specific benefits provided by these compositions is that they provide sustained effective and tolerable plasma levels with a regimen of only one or two dosings per day.

For the avoidance of doubt, the respective treatment durations do not require that the same dosing regimen is used throughout the entire treatment. For example, if once-daily dosing becomes insufficient to treat a specific subject, the regimen may be switched to twice daily administration. Alternatively, the dose per administration event may be increased within chronic treatment.

The following diseases or conditions may be considered as responsive, or at least potentially responsive, to bradykinin B2 receptor modulation:
Skin disorders include, without limitation, disorders such as skin aging, skin efflorescences including pressure sores, decubital ulcers, irritated, sensitive and dysaesthetic skin, erythema, rash, skin edema, psoriasis, eczema, lichen, bacterial, viral, fungal and parasites induced skin infections including furuncle, abscess, phlegmon, erysipelas, folliculitis and impetigo, lice, scabies and herpes simplex, acne, exanthema, dermatitis including atopic dermatitis, allergic contact dermatitis, neurodermatitis, radiation damage, sunburn, pruritus, itching, urticaria, psoriasis, mycosis, tissue ulceration, epidermolysis bullosa, wounds including abnormal wound healing, burns, frostbite, skin inflammation and edema caused by venoms, alopecia, hair squama, corn, wart and panaris.

Eye diseases include, without limitation, inflammatory disorders such as scleritis, conjunctivitis, chemosis, iritis, iridocyclitis, uveitis, chorioretinitis, as well as disorders such as retinochoroidal circulatory disorders, bacterial eye infections, unspecific conjunctivitis and eye irritations, retinopathy of prematurity, proliferative vitreoretinopathy, macular degeneration (including age related macular degeneration and including both wet and dry forms), corneal diseases including corneal graft rejection, corneal injury, corneal scarring, corneal ulceration, corneal haze, keratoconus, glaucoma (preferably open angle glaucoma), myopia, ocular hypertension, ocular vessel damage, angiogenesis, eye fibrosis (e.g. anterior subcapsular fibrosis, posterior subcapsular opacities, posterior capsular opacities, corneal haze after laser surgery, subconjunctival scarring after glaucoma surgery), proliferative vitreoretinopathy (PVR), bacterial ocular infections including hordeolum and ptilosis.

Ear diseases encompass, but are not limited to, disorders such as Meniere's disease, inflammation of the middle ear, inflammation of the external auditory canal and acute hearing loss.

Mouth, throat and respiratory diseases comprise, without limitation, disorders such as inflammation of the oral mucosa and gums including aphta and stomatitis, parodontitis, epiglottitis, pharyngitis, laryngotracheitis, tonsillitis, common cold, angina, rhinitis including seasonal allergic rhinitis or perennial allergic rhinitis, rhinorrea, sinusitis of whatever type, etiology or pathogenesis or sinusitis that is a member selected from the group consisting of purulent or nonpurulent sinusitis, acute and chronic sinusitis and ethmoid, frontal, maxillary or sphenoid sinusitis, expectoration, pneumoconiosis of whatever type or genesis, including for example aluminosis, anthracosis, asbestosis, chalicosis, siderosis, silicosis, tabacosis and, in particular, byssinosis, bronchitis, cough, trachitis, congestion, pneumonia, eosinophilc lung infiltrate, chronic eosinophilic pneumonia, idiopathic pulmonary fibrosis and other fibrotic lung diseases, treatment related fibrotic lung disease e.g. related to radiation, methotrexate, chemotherapy, amiodarone or nitrofurantoin, sarcoidosis, acute respiratory distress syndrome (ARDS), bronchoconstriction, asthma of whatever type, etiology, or pathogenesis, or asthma that is a member selected from the group of atopic asthma, non-atopic asthma, allergic and non-allergic asthma, extrinsic asthma caused by environmental factors, intrinsic asthma caused by pathophysiologic disturbances, bronchial asthma, IgE-mediated asthma, essential asthma and essential asthma of unknown or inapparent cause, true asthma, emphysematous asthma, exercise-induced asthma, occupational asthma, infective asthma caused by bacterial, fungal, protozoal or viral infection, incipient asthma, wheezy infant syndrome, bronchial hyperreactivity, chronic obstructive pulmonary disease (COPD), COPD that is characterized by irreversible, progressive airways obstruction, acute respiratory distress syndrome (ARDS) and exacerbation of airways hyperreactivity consequent to other drug therapy, dyspnea, hyperoxic alveolar injury, pulmonary emphysema, pleurisy, tuberculosis, exposure to high altitude i.e. acute mountain sickness and preferably high altitude pulmonary edema (HAPE), resistant cough, bronchial hyporeactivity.

Gastrointestinal diseases include, without limitation, disorders including esophagitis, gastritis, irritable stomach, gastric and duodenal ulcer, ileus, colon irritable, inflammatory bowel diseases including Crohn's disease and ulcerative colitis, enteritis, hypertensive gastro- and colopathy, colitis, peritonitis, appendicitis, rectitis, gastrointestinal hemorrhage caused by a portal hypertension, collateral circulation or hyperemia, postgastrectomy dumping-syndrome, digestion discomfort, diarrhea, hemorrhoids, worm diseases, abdominal colic and colic of parts of the gastrointestinal system.

Liver, gallbladder and pancreatic diseases encompass, but are not limited to, disorders such as hepatitis, cirrhosis of the liver, liver fibrosis (e.g. due to viral (HBV/HCV) infections, toxins (alcohol), fatty liver, bile stasis, hypoxia), portal hypertension, hepatorenal syndrome, hepatogenic edema, cholangitis, cholecystitis, acute and chronic pancreatitis, and biliary colic.

Urinary tract and kidney diseases include, without limitation, urinary tract infections such as acute and chronic cystitis, interstitial cystitis, irritable bladder, overactive bladder, incontinence including but not limited to stress-, urge and reflex incontinence, benign prostate hyperplasia, chronic renal disease, urethritis, inflammatory kidney diseases including glomerulonephritis, glomerular disease of the kidney, interstitial nephritis, pyelonephritis, diuresis, proteinuria, natriuresis, calciuresis, disorders of water balance, disorders of electrolyte balance, disorders of acid-base balance and renal colic, renal fibrosis, chronic renal allograft dysfunction, contrast-induced nephropathy.

Diseases of male genital organs and female genital organs include, without limitation, altered sperm mobility, male infertility, orchitis, prostatitis, prostate enhancement, mastitis, inflammatory pelvis diseases, vaginal infections and pain, adnexitis, colpitis, soft ulcus, syphilis, clap and ovarian hyperstimulation syndrome.

Diseases of the hormone system include, without limitation, menstrual disorders and pain, climacteric disturbance, emesis, premature uterine contractions, premature labor, endometriosis, endometritis, myoma, pre-eclampsia.

Metabolic diseases include, without limitation, disorders such as diabetes, including non-insulin dependent diabetes mellitus, diabetic retinopathy, diabetic macular edema, diabetic nephropathy and diabetic neuropathy, insulin resistance and diabetic ulceration, diseases of the proteo- and purine metabolism such as gout and disorder of lipometabolism, hypoglycemia.

Cardiovascular diseases include, without limitation, disorders including vascular permeability, vasodilation, peripheral circulatory disorders, arterial circulatory disorders including aortic aneurysm, abdominal aortic aneurysm, brain aortic aneurysm, hypertension and hypotension associated with sepsis, restenosis after percutaneous transluminal coronary angioplasty, atherosclerosis including atherosclerotic plaque rupture, hemangioma, angiofibroma, venous disorders such as thrombosis, varicosity, phlebitis, thrombophlebitis, phlebothrombosis, cardiopathy, congestive heart failure, coronary heart disease, carcinoid syndrome, angina pectoris, cardiac dysrhythmias, inflammatory heart diseases including endocarditis, pericarditis and constrictive pericarditis, myocarditis, myocardial infarct, postmyocardial infarction syndrome, left ventricular dilation, post ischemic reperfusion injury, shock and collapse including septic, allergic, post traumatic and hemodynamic shock, amniotic fluid embolism, systemic inflammatory response syndrome (SIRS) including SIRS caused by heartlung bypass during surgery, sepsis and internal and external complications during cardiopulmonal bypass surgery (including but not limited to adverse hemodynamic effects following protamine sulfate reversal of heparin.

Blood diseases include, without limitation, disorders such as coagulation, disseminated intravascular coagulopathy, hemorrhage, hemorrhagic diathesis, hypercholesterolemia and hyperlipemia, hypovolemic shock, paroxysmal nocturnal haemoglobinuria.

Lymphatic diseases include, without limitation, splenomegaly, lymphangitis, lymphadenitis and hyperplastic adenoids.

Disorders of the central nervous system include, without limitation, disorders such as inflammatory diseases of the central nervous system including encephalitis, meningitis, encephalomyelitis, meningoencephalitis, hydrocephalus, amyotrophic lateral sclerosis, spinal cord trauma, spinal cord edema, demyelinating diseases of the nervous system, multiple sclerosis, acute and chronic neuro-degenerative disorders including aging, Alzheimer's disease and Parkinson's disease, neuritis, and peripheral neuropathy, depressions, anorexia, anxiety and schizophrenia, sleep disorders.

Brain disorders include, without limitation, disorders such as nootropic or cognition enhancement, cerebral amyloid angiopathy, stroke, head and brain trauma, traumatic brain injury, brain tumor, cerebral heat damage, cerebral ischemia, cerebral hemorrhage, post traumatic and post ischemic cerebral edema, general brain edema, acute mountain sickness and preferably high altitude cerebral edema (HACE), cytotoxic brain edema, vasogenic brain edema, post-surgical brain edema, brain edema associated with metabolic diseases, increase of permeability of blood-brain barrier or blood-brain tumor barrier.

Musculoskeletal system diseases include, without limitation, disorders such as inflammatory musculoskeletal disorders, arthrosis, osteoarthrosis, osteoarthritis, chondroporosis after joint trauma or relatively long immobilization of a joint after meniscus or patella injuries or torn ligaments, rheumatoid arthritis of whatever type, etiology, or pathogenesis including acute arthritis, acute gouty arthritis, chronic inflammatory arthritis, degenerative arthritis, infectious arthritis, Lyme arthritis, proliferative arthritis, vertebral arthritis, septic arthritis, psoriatic arthritis, chronic polyarthritis, rheumatism, Sjogren's syndrome, lumbago, spondylitis, spondylarthritis, ankylosing spondylitis, osteomyelitis, sprain, teno-synovitis, inflammation-induced bone resorption, fracture or the like, osteoporosis, musculoskeletal pain and hardening, spinal disk syndrome.

Allergy disorders include, without limitation, disorders such as general allergic reactions, food allergy, anaphylactic shock, allergic contact hypersensitivity, allergic skin reactions, allergic asthma, vernal conjunctivitis and seasonal or perennial allergic rhinitis.

Pain includes, without limitation, centrally and peripherally mediated pain, vascular pain, visceral pain, inflammatory mediated pain, neuralgic pain, referred pain, nociceptive pain, reflectory pain, psychosomatic pain, acute pain such as caused by acute injury, trauma or surgery of bones, muscle, tissue, soft tissue, organs, pain after insect bites, post-stroke pain syndrome, post-surgery pain, progressive disease related pain, chronic pain such as caused by neuropathic pain conditions (including but not limited to complex regional pain syndrome, causalgia, morbus sudeck, reflex sympathetic dystrophy, diabetic peripheral neuropathy, post-herpetic neuralgia, trigeminal neuralgia, cancer-related pain, pain associated with rheumatoid arthritis, osteoarthritis, teno-synovitis, gout, menstruation and angina, fibromyalgia, ocular pain, back pain, headache, cluster headache, migraine, inflammatory pain, which may be associated with acute inflammation or chronic inflammation. Inflammatory pain includes but is not limited to neuropathic pain, ischemic pain, pain induced by arthritis, muscle pain induced by acute or chronic inflammation, neuralgia caused by acute or chronic inflammation, hyperalgesia. Also chemotherapy-induced peripheral neuropathy, hyperalgesia, opioid-induced hyperalgesia and fever. Furthermore, compounds of the invention are useful as analgesic agent for use during general and monitored anesthesia.

Infectious diseases include, without limitation, diseases including those mediated by bacteria, viruses, fungi, parasites, protozoa, prions or mycobacterial infections. Particularly, the present invention is useful for the treatment of bacterial infections caused by Streptococcus, Escherichia, Salmonella, Staphylococcus, Klebsiella, Moracella, Haemophilus and Yersinia. Examples of bacterial infections intended to be within the scope of the present invention include, but are not limited to diseases such as pestis, sepsis, epidemic typhus, food poisoning, tetanus, scarlet red, whooping cough, diphtheria. Examples of viral infections intended to be within the scope of the present invention include, but are not limited to diseases such chickenpox and herpes zoster, AIDS, influenza, small pox, and children diseases such as measles, rubella, mumps, acute anterior poliomyelitis. The present invention is useful for the treatment of protozoa and parasites infections caused by Schistosoma mansoni, Dermatofagoides farinae, and Plasmodium inducing Malaria. Examples of prion infections intended to be within the scope of the present invention include, but are not limited to diseases such bovine spongiform encephalopathy (BSE), Creutzfeldt Jacob disease and kuru, dengue fever, hemorrhagic fever.

Inflammatory disorders include, without limitation, disorders such as acute-phase reaction, local and systemic inflammation and inflammation caused by other diseases whatever type, etiology or pathogenesis and caused by those inflammatory diseases specified within this application.

Injuries: Within the present application the term "injuries" encompasses, but is not limited to, multiple trauma, head trauma, lung injuries, external, internal and surgery wounds.

Immunology disorders include, without limitation, disorders such as hyperesthesia, autoimmune disorders, graft rejection in transplantation, transplant toxicity, granulomatous inflammation / tissue remodelling, myasthenia gravis, immunosuppression, immune-complex diseases, over- and underproduction of antibodies, vasculitis, delayed graft function, lupus.

Cancers include, without limitation, disorders such as solid tumor cancer including breast cancer, lung cancer (non-small-cell lung cancer and small-cell lung cancer), prostate cancer, cancers of the oral cavity and pharynx (lip, tongue, mouth, pharynx), esophagus, stomach, small intestine, large intestine, colon, rectum, gallbladder and biliary passages, pancreas, larynx, lung, bone, osteosarcoma, connective tissue, skin cancer including Kaposi's syndrome, melanoma and skin metastasis, epidermoid cancer, basal cell carcinoma, cervix uteri, corpus endometrium, cancer of ovary, testis, bladder, ureter and urethra, kidney, eye, brain and central nervous system, pseudotumor cerebri, sarcoma, sarcoid, thyroid and other endocrine glands (including but not limited to carcinoid tumors), Hodgkin's disease, non-Hodgkin's lymphomas, multiple myeloma, hematopoetic malignancies including leukemias and lymphomas including lymphocytic, granulocytic and monocytic lymphomas, tumor invasion, metastasis, ascites, tumor growth and angiogenesis.

Hereditary diseases include, without limitation, disorders such as hereditary angioedema and angioneurotic edema, chondrocalcinosis, Huntington's disease, mucoviscidosis.

Edema, as used herein, includes, but is not limited to, general edema and edema caused by inflammation, Factor XII deficiency-induced edema, other drugs, e.g. drug induced angioedema, including but not limited to angiotensin-converting enzyme inhibitor-induced angioedema, infection, burns, injuries, trauma, frostbite, surgery, distorsions, fractures, exposure to high altitude (e.g. high altitude pulmonary edema (HAPE) and high altitude cerebral edema (HACE)), hereditary, autoimmune and other diseases and disorders, particularly but not limited to those disorders specified in this application, stress-induced edema (pronounced swelling) of gut. Known forms of angioedema (AE) include hereditary angioedema (HAE), acquired angioedema (AAE), bradykinin-mediated non-histaminergic idiopathic angioedema, allergic angioedema, and drug induced angioedema. The HAE can be of any type, including type I HAE, type II HAE, or type III HAE.

Capillary leak syndrome(s) include(s), without limitation, systemic capillary leak syndrome in sepsis, burn, allergy, drug/toxin-induced conditions, organ transplantation and IL-2 cytokine therapy.

In a further preferred embodiment, the matrix tablet, in particular the extended-release matrix tablet according to any of the preferred embodiments as disclosed herein, is used in the treatment of AE, including HAE, AAE, bradykinin-mediated non-histaminergic idiopathic AE, allergic AE, and drug induced AE. AE is an area of swelling of the lower layer of skin and tissue just under the skin or mucous membranes. The debilitating and often painful swelling may occur in the face, lips, tongue, limbs, genitals, gastrointestinal mucosa, urogenital region and airways. Often it is associated with hives, which are swelling within the upper skin. It is characterized by repetitive episodes of swelling, and onset is typically over minutes to hours. Predicting where and when the next episode of angioedema will occur is impossible. Patients may have one episode per month, but there are also patients who have weekly episodes or only one or two episodes per year.

In some preferred embodiments, the matrix tablet, in particular the extended-release matrix tablet according to any of the preferred embodiments as disclosed herein, is used in the treatment of HAE of any type, including type I HAE, type II HAE, or type III HAE, preferably type I HAE or type II HAE. HAE is a disorder that manifests in recurring attacks of severe swelling. The swelling often affects the arms, legs, face, intestinal tract, and also the airways. If the intestinal tract is affected, abdominal pain and vomiting may occur. Swelling of the airways may result in its bronchial obstruction and breathing difficulties. Acute attacks typically last for several days, and HAE patients experience attacks at a frequency of about every two weeks.

The matrix tablet is particularly advantageous in the chronic therapy of AE, including hereditary angioedema (HAE) and acquired angioedema (AAE), and in particular type I HAE or type II HAE. As mentioned, it is patient-friendly in that it only requires the administration of a single daily dose or, alternatively, only two daily doses. In view of the robustness of the extended-release profiles with respect to pH-variations and the presence of food, these compositions are suitable for long-term therapy over periods of months or even years. Accordingly, one of the preferred embodiments is directed to the use of the matrix tablet, in particular the extended-release matrix tablet according to any of the preferred embodiments as disclosed herein, for the chronic treatment of hereditary angioedema, wherein the chronic treatment is preferably conducted over a period of at least one month. As previously mentioned, the chronic treatment may also be conducted over at least 3 months, or over at least one year, respectively. Further preferred is the chronic treatment using a dosing regimen comprising once-daily or twice-daily administration of the matrix tablet.

**In** other words, and with respect to the BK B2 receptor antagonist of Formula (1), the matrix tablet design as described herein enables prolonged release profiles that will allow longer dosing intervals, which is associated with increased patient friendliness, leading to the patients' better adherence to the prescribed dosing regimen, and thus to improved therapeutic outcome.

Again, for the avoidance of doubt, the respective treatment durations do not require that the same dosing regimen is used throughout the entire treatment. For example, if once-daily dosing becomes insufficient to treat a specific subject, the regimen may be switched to twice daily administration. Alternatively, the dose of the BK B2 receptor antagonist per administration event may be increased within chronic treatment.

### Items of the invention

The invention generally relates to the following items:
1. A pharmaceutical matrix tablet comprising a bradykinin (BK) B2 receptor antagonist having a chemical structure according to Formula (1), or a stereoisomer, salt or solvate thereof: 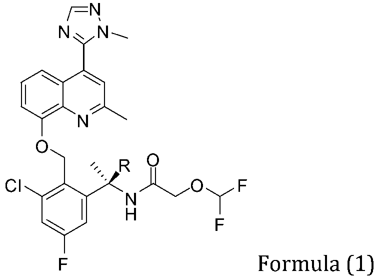 wherein R is deuterium or hydrogen; and at least one hydrophilic matrix-forming polymer.
2. The pharmaceutical matrix tablet of item 1, wherein the BK B2 receptor antagonist is a compound according to Formula (1) or a solvate thereof, and wherein R is deuterium.
3. The pharmaceutical matrix tablet of item 1 or 2, consisting of an optionally coated single-layer tablet.
4. The pharmaceutical matrix tablet of any one of the preceding items, wherein the matrix tablet is an extended-release matrix tablet.
5. The pharmaceutical matrix tablet of any one of the preceding items, wherein the hydrophilic matrix-forming polymer is selected from hydroxypropyl cellulose, hydroxypropyl methylcellulose, hydroxypropyl methylcellulose acetate succinate, alginic acid, bentonite, carbomer, carboxymethylcellulose calcium, carboxymethyl cellulose sodium, carrageenan, cellulose, copovidone, gelatin, guar gum, hydroxyethylcellulose, hydroxypropyl starch, methylcellulose, pectin, polyethylene oxide, polymethacrylates, potassium (or calcium, or sodium) alginate, povidone, tragacanth, or xanthan gum.
6. The pharmaceutical matrix tablet of item 5, wherein the hydrophilic matrix-forming polymer is selected from hydroxypropyl cellulose, hydroxypropyl methylcellulose, and combinations thereof.
7. The pharmaceutical matrix tablet of item 6, wherein the hydrophilic matrix-forming polymer comprises, or consists of, a combination of a hydroxypropyl methylcellulose type 2208 and a hydroxypropyl methylcellulose type 2910.
8. The pharmaceutical matrix tablet of item 7, comprising 20 wt.% to 40 wt.% of hydroxypropyl methylcellulose, or of a combination of different grades of hydroxypropyl methylcellulose.
9. The pharmaceutical matrix tablet of any one of the preceding items, further comprising a tablet filler selected from microcrystalline cellulose and/or calcium hydrogen phosphate dihydrate.
10. The pharmaceutical matrix tablet of item 9, wherein the microcrystalline cellulose is silicified.
11. The pharmaceutical matrix tablet of any one of the preceding items, wherein the BK B2 receptor antagonist is incorporated in micronised form.
12. The pharmaceutical matrix tablet of any one of the preceding items, wherein the BK B2 receptor antagonist is incorporated in solubilised form.
13. The pharmaceutical matrix tablet of item 12, wherein the BK B2 receptor antagonist is incorporated predominantly in the amorphous form.
14. The pharmaceutical matrix tablet of item 12 or 13, comprising a solid dispersion of the amorphous BK B2 receptor antagonist in a carrier, wherein the carrier comprises at least one pharmaceutically acceptable carrier polymer selected from hydroxypropyl methylcellulose, hydroxypropyl methylcellulose acetate succinate, copovidone and combinations thereof.
15. The pharmaceutical matrix tablet of item 14, wherein the solid dispersion is incorporated as particles having an average particle size D50 of not more than 250 µm.
16. The pharmaceutical matrix tablet of item 14 or 15, wherein the solid dispersion comprises at least 10 wt.% of BK B2 receptor antagonist, relative to the total weight of the solid dispersion.
17. The pharmaceutical matrix tablet of any one of items 14 to 16, wherein the matrix tablet comprises 15 wt.% to 40 wt.% of the solid dispersion, relative to the total weight of the tablet without its optional coating.
18. The pharmaceutical matrix tablet of any one of items 14 to 17, wherein the weight ratio of the solid dispersion to the hydrophilic matrix-forming polymer is in the range from 2 : 1 to 1 : 2.
19. The pharmaceutical matrix tablet of any one of items 14 to 18, comprising:
   - 15 wt.% to 40 wt.% of the solid dispersion,
   - 20 wt.% to 40 wt.% of the hydrophilic matrix-forming polymer, and
   - 30 wt.% to 50 wt.% of the filler.
20. The pharmaceutical matrix tablet of item 19, comprising:
   - 20 wt.% to 40 wt.% of the solid dispersion,
   - 20 wt.% to 40 wt.% of hydroxypropyl methylcellulose, or of a combination of different grades of hydroxypropyl methylcellulose,
   - 30 wt.% to 50 wt.% of microcrystalline cellulose, calcium hydrogen phosphate dihydrate, or a combination thereof,
   - up to 2 wt.% of a glidant, in particular colloidal silicon dioxide, and
   - up to 2 wt.% of a lubricant, in particular magnesium stearate.
21. The pharmaceutical matrix tablet of any one of the preceding items, comprising a coating, said coating comprising a water-soluble film-forming polymer and a plasticiser.
22. The pharmaceutical matrix tablet of item 21, wherein the amount of the coating is 2 wt.% to 8 wt.% relative to the weight of the uncoated tablet.
23. The pharmaceutical matrix tablet of any one of the preceding items, wherein the matrix tablet releases the BK B2 receptor antagonist over a period of at least 8 hours, as determined by dissolution testing according to USP chapter <711>, using apparatus II, at pH 6.8 and a paddle rotation speed of 100 rpm.
24. The pharmaceutical matrix tablet of item 23, wherein the matrix tablet releases the BK B2 receptor antagonist over a period of 12 to 24 hours.
25. A method for preparing the matrix tablet of any one of the preceding items, comprising the steps of:
   (a) providing a solid dispersion in powder or granular form, the solid dispersion comprising BK B2 receptor antagonist and a carrier comprising at least one pharmaceutically acceptable carrier polymer, wherein the BK B2 receptor antagonist is amorphous and dispersed in the carrier;
   (b) combining the solid dispersion with a hydrophilic matrix-forming polymer, a filler and optionally further excipients such as to form a compressible powder or granule mixture, and
   (c) compressing the powder or granule mixture to obtain the matrix tablet.
26. The method of item 25, further characterised in that step (c) of compressing the powder or granule mixture to obtain the matrix tablet is conducted without any prior agglomeration step.
27. The pharmaceutical matrix tablet of any one items 1 to 24 for use in the treatment of a subject suffering from a disease or condition responsive to BK B2 receptor modulation.
28. The pharmaceutical matrix tablet for use according to item 27, wherein the disease or condition responsive to BK B2 receptor modulation is selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; edema; and capillary leak syndrome.
29. The pharmaceutical matrix tablet for use according to item 27 or 28, wherein the disease or condition responsive to BK B2 receptor modulation is angioedema, such as hereditary angioedema (HAE) or acquired angioedema (AAE).
30. The pharmaceutical matrix tablet for use according to any one of items 27 to 29, wherein the treatment is chronic treatment over at least one month.
31. A method of treating or preventing a disease or condition that is responsive to BK B2 receptor modulation in a subject, comprising orally administering to the subject with a disease or condition responsive to BK B2 receptor modulation an effective amount of the pharmaceutical matrix tablet of any one of the items 1 to 24 thereby preventing or treating said condition or disease in said subject.
32. The method of item 31, wherein the disease or condition that is responsive to BK B2 receptor modulation is selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; edema; and capillary leak syndrome.
33. The method of item 31 or 32, wherein the disease or condition that is responsive to BK B2 receptor modulation is angioedema, such as hereditary angioedema (HAE) or acquired angioedema (AAE).
34. The method of any one of the items 31 to 33 which comprises repeated administration of the pharmaceutical matrix tablet according to a regular dosing regimen over an extended period of time for chronic treatment.
35. Use of the pharmaceutical matrix tablet of any one of the items 1 to 24 in the manufacture of a medicament for treating or preventing a disease or condition that is responsive to BK B2 receptor modulation.
36. The use of item 35, wherein the disease or condition that is responsive to BK B2 receptor modulation is selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; edema; and capillary leak syndrome.
37. The use of item 35 or 36, wherein the disease or condition that is responsive to BK B2 receptor modulation is angioedema, such as hereditary angioedema (HAE) or acquired angioedema (AAE).
38. The use of any one of the items 35 to 37, wherein the medicament is intended for chronic treatment.

Further aspects of the invention, embodiments, optional features and preferences will become clear on the basis of the Examples and the patent claims.

### Further definitions

For clarity, some further definitions of terms are given which are used throughout the description and claims. The definitions should be used to determine the meaning of the respective expressions unless the context requires a different meaning.

The terms 'a' or 'an' do not exclude a plurality, i.e., the singular forms 'a', 'an' and 'the' should be understood as to include plural referents unless the context clearly indicates or requires otherwise. In other words, all references to singular characteristics or limitations of the present disclosure shall include the corresponding plural characteristic or limitation, and vice versa, unless explicitly specified otherwise or clearly implied to the contrary by the context in which the reference is made. The terms 'a', 'an' and 'the' hence have the same meaning as 'at least one' or as 'one or more' unless defined otherwise. For example, reference to 'an ingredient' includes mixtures of ingredients, and the like.

The terms 'about' or 'ca.' will compensate for variability allowed for in the pharmaceutical industry and inherent in pharmaceutical products, such as differences in content due to manufacturing variation and/or time-induced product degradation. The term allows for any variation, which in the practice of pharmaceuticals would allow the product being evaluated to be considered bioequivalent in a subject to the recited strength of a claimed product.

The terms 'active agent', 'therapeutic agent', 'active pharmaceutical ingredient (API)', 'active principle', 'drug', 'bioactive agent', are used synonymously and refer to a compound or combination of compounds which are pharmaceutically active against an undesired condition.

The term 'composition' refers to any type of composition in which the specified ingredients may be incorporated, optionally along with any further constituents.

A "pharmaceutical composition", in the context of the present invention, should be understood as a composition that is technically suitable for being administered as a medicament to a subject, such as a human patient. It is composed, formulated and processed in compliance with general pharmaceutical standards, as may be defined for example in the official pharmacopoeias or guidance issued by the regulatory agencies such as the FDA, PMDA and the EMA. In one of the preferred embodiments, the composition is adapted for oral administration, which implies, for example, that the excipients used, including their grades and their amounts, are safe and acceptable for oral use, in particular for oral administration to a human subject.

An "extended-release composition", as used herein, is any pharmaceutical composition or formulation adapted for oral administration which exhibits extended-release of the active ingredient. Extended-release may also be referred to as slow release, controlled release, or sustained release, to mention only a few alternative expressions, without limitation.

The term 'compound' refers to a chemical substance, which is a material consisting of molecules having essentially the same chemical structure and properties. For a small molecular compound, the molecules are typically identical with respect to their atomic composition and structural configuration. For a macromolecular or polymeric compound, the molecules of a compound are highly similar but not all of them are necessarily identical.

The terms 'comprise', 'comprises' and 'comprising' and similar expressions are to be construed in an open and inclusive sense, as 'including, but not limited to'.

The terms 'essentially', 'about', 'approximately', 'substantially' and the like in connection with an attribute or value include the exact attribute or the precise value, as well as any attribute or value typically considered to fall within a normal range or variability accepted in the technical field concerned. For example, 'essentially free of water' means that no water is deliberately included in a composition but does not exclude the presence of residual moisture.

The term 'essentially consisting of refers to compositions or dosage forms where no further components are added other than those listed. Nevertheless, very small amounts of other materials may potentially be present, such as material inherent impurities, or minor additives added by excipient providers. Furthermore, when referring to e.g., 'essentially consisting of A, B, C and optionally D.' this means that no further components are added to a composition or dosage form other than A, B, C and D, with D being an optional component (i.e., not mandatory) in said composition or dosage form.

The term 'essentially free' refers to a composition that contains less than a functional amount of the respective ingredient, typically less than 1 % by weight, preferably less than 0.1 % or even 0.01 %, and including zero percent by weight of the respective ingredient.

Some aspects of the invention are further illustrated by the following Examples, which should however not be understood as restricting the scope of the invention.

### EXAMPLES

The compound of Formula (1) was prepared as described in WO 2019/101906 and, where necessary, under Good Manufacturing Practice (GMP) regulations.

The study reported in Example 6 was carried out in accordance with corresponding protocols, current guidelines on Good Clinical Practice (GCP) of the International Council for Harmonisation of Technical Requirements for Pharmaceuticals for Human Use (ICH), and applicable regulatory and country-specific governmental rules for conducting clinical trials, including rules on the protection of subjects and informed consent, and transparency requirements. All trial protocols, suitability of the investigator(s), facilities and the methods and material to be used in obtaining and documenting informed consent of the trial subject were reviewed and approved by the Institutional Review Board (IRB) or Independent Ethics Committee (IEC) before a study was started. For this study, a qualified person performed the final release of the study drug, i.e. the compound of formula (I), according to Directive 2003/94/EC annex 13; and study drug labels contained information to meet the applicable regulatory requirements. Trial medication was packed, labelled and released under the responsibility of the pharmacist of the clinical site in accordance with GMP practice guidelines, International Conference on Harmonization (ICH), Good Clinical Practice (GCP) and applicable local laws/regulations.

### Example 1: Solid dispersions prepared by vacuum melt compression

Two solid dispersion compositions were prepared by vacuum melt compression. The first one, designated as HME1, consisted of 40 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, 58.5 wt.% copovidone (Kollidon^{®} VA64), and 1.5 wt.% of poloxamer 188. The second composition, designated as HME2, consisted of 20 wt.% (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and 80 wt.% of hypromellose acetate succinate (AQOAT^{®} AS-MMP).

The active ingredient, (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide, was provided as crystalline monohydrate in powder form. The measured amounts of the active ingredient and the carrier were mixed for 4 minutes in a shaker mixer (Turbula^{®}).

Subsequently, measured amounts of the powder mixtures were transferred into a lab-scale vacuum compression moulding apparatus (VCM Meltprep) equipped with a disc moulding tool (Meltprep Disc Tool) and moulded into discs at a vacuum of -1.0 bar. For HME1, the vacuum compression moulding occurred at a temperature of 200 °C, using a heating time of 5 min, a cooling time of 2 min and a cooling air pressure of 2.5 bar. For HME2, the vacuum compression moulding occurred at a temperature of 180 °C, using a heating time of 5 min, a cooling time of 5 min and a cooling air pressure of 3.0 bar. With both compositions, transparent yellowish discs were obtained.

The discs were crushed with a mortar and a pestle for further assessment. Firstly, it was confirmed by an HPLC assay that the drug content was within the range of 90 to 110 % of the theoretical amount. Moreover, the crystalline state of the drug substance was assessed by differential scanning calorimetry (DSC) and X-ray powder diffraction (XRPD, also referred to as powder X-ray diffraction or PXRD). The DSC thermograms showed no melting point of the active ingredient (around 111°C), only the Tg of the polymers were observable. The XRPD analysis indicated no residual crystallinity. Therefore it was concluded that the samples represented solid dispersions comprising the active ingredient solely in the amorphous, fully dissolved state.

### Example 2: Solid dispersions prepared by spray drying

Four solid dispersion compositions, designated as SDD1 through SDD4, were prepared by spray drying, each consisting of 25 wt.% of (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and 25 wt.% of carrier polymer. The carrier polymers were hypromellose acetate succinate (AQOAT^{®} AS-MMP) for SDD1, hypromellose of type 2910 USP (Methocel^{™} E3) for SDD2, copovidone (Kollidon^{®} VA64) for SDD3, and povidone K-30 for SDD4, respectively.

For each composition, the measured amounts of the active ingredient and the respective carrier polymer were dissolved in a mixture of dichloromethane (DCM) and methanol (ratio 4 : 1 (w/w)). Spray drying was then performed using a lab-scale spray dryer (Büchi B290), using the parameters listed in Table 1 below.

**Table 1**

| Parameter | Value |
|---|---|
| Nozzle | 2-Fluid nozzle, 1.5 mm cap, 0.7 mm tip |
| Glassware | High efficiency cyclone |
| Atomizing air flow rate | 40 L/min |
| Solution feed | 7-12 g/min |
| Inlet temperature | 57-58 °C |
| Outlet temperature | 35-36 °C |
| Blower (aspirator) | 100% |
| Wash solution | DCM/methanol (4 : 1 v/v) |

The spray drying process yielded powders that were collected and subjected to a secondary drying step, using vacuum drying at 40 °C and a vacuum of -26.5 mm Hg over 20 hours.

The samples thus obtained were assessed using modulated differential scanning calorimetry (mDSC), XRPD, and thermogravimetric analysis (also thermal gravimetric analysis, TGA). Both XRPD and mDSC data confirmed that the four prototypes were amorphous and free of any detectable levels of crystalline material. The TGA thermogram showed an inflection in the slope of the weight loss step for the SDD4 prototype, indicating a small amount of residual solvent left in the sample. Overall, all prototypes were confirmed to represent homogenous solid dispersions comprising the active ingredient in amorphous form, and fully dissolved in the carrier polymer.

Samples of the spray dried solid dispersions SSD1 through SSD4 were stored at elevated temperature (40 °C) and retested by XRPD after three weeks. In result, all samples were amorphous and free of crystalline content.

### Example 3: Solid dispersion prepared by hot melt extrusion

The same composition as HME2, consisting essentially of 20 wt.% (S)-N-(1-deutero-1-(3-chloro-5-fluoro-2-((2-methyl-4-(1-methyl-1H-1,2,4-triazol-5-yl)quinolin-8-yloxy)methyl)phenyl)ethyl)-2-(difluoromethoxy)acetamide and 80 wt.% of hypromellose acetate succinate (AQOATO AS-MMP) (see Example 1), was used for preparing a solid dispersion by hot melt extrusion. The powder blend comprising the crystalline active ingredient and the carrier polymer was screened through a sieve having an aperture width of 500 µm. The blend was then extruded using a twin screw extruder (Leistritz ZSE 12 HP-PH) fitted with a 1.5 mm die. The maximum temperature of the kneading zones was within a range of 145 to 165 °C, except that the die zone was set at 200 °C. Clear extrudates of a slightly brownish colour were obtained and pelletised with a pelletiser. Subsequently, the pellets were milled using a hammer mill in two phases, the first one with a screen size of 1,000 µm, the second one with a screen size of 400 µm, at a mill speed of 6,500 rpm. The particle size distribution was then characterised by laser diffraction (Mastersizer^{®}). For two milled batches, the average particle size D50 was 262.3 µm and 249.1 µm, respectively. In a sieve analysis, it was found that 66.2 wt.% and 61.7 wt.% of the milled particles, respectively, were in the sieve fractions of 180 µm to 355 µm.

Samples of the HME2 solid dispersion were stored at accelerated temperature (40 °C) to test their chemical and physical stability. The drug content and the impurities were quantified by HPLC. XRPD was used to detect changes in the crystalline state, if any. The test results are provided in Table 2 below. They indicate that the active ingredient is chemically highly stable in the solid dispersion and remains in the amorphous form.

**Table 2**

| Time | Start | 3 months | 6 months | 6 months |
|---|---|---|---|---|
| Temperature | - | 40 °C | 25 °C | 40 °C |
| Assay* (wt.%) | 98.5 | 99.5 | 98.4 | 98.3 |
| Total impurities (wt.%) | 0.85 | 0.89 | 0.94 | 0.93 |
| Crystallinity | None | None | None | None |

| | | | | |
|---|---|---|---|---|
| *100% means the theoretical amount of the API in the sample | | | | |

### Example 4: Matrix tablets comprising micronised active ingredient

About 2.9 kg of crystalline (*S*)-N-(1-deutero-1-(3-chloro-5-fluoro-2-((2-methyl-4-(1-methyl-1*H*-1,2,4-triazol-5-yl)quinolin-8-yloxy)methyl)phenyl)ethyl)-2-(difluoromethoxy)acetamide monohydrate was micronised in a jet mill using nitrogen as process gas, at a gas feed pressure of about 30 psi for the milling chamber and of about 60 psi for the venturi injector. A micronised powder was obtained, having a particle size distribution characterised by a D50 of 5.66 µm, a D90 of 11.70 µm, and a D10 of 1.40 µm, based on measurement by laser diffraction. The specific surface area of the powder was 2.19 m²/g.

In a next step, five different types of extended-release matrix tablets incorporating the micronised API were prepared. The composition of these tablets is provided in Table 3 below. The tablets were compressed from the respective powder mixtures on a lab-scale, single-punch pharmaceutical tablet press (Kilian Styl'One Evolution) using a precompression force of 0.8 to 2.3 kN and a main compression force of 13 to 25 kN. In all cases, tablets having a mean resistance to crushing of more than 100 N were obtained.

**Table 3**

| Components per tablet (mg) | 4.1 | 4.2 | 4.3 | 4.4 | 4.5 |
|---|---|---|---|---|---|
| Micronised API | 20.0 | 20.0 | 20.0 | 20.0 | 20.0 |
| Hypromellose type 2208 (Methocel^{™} K15M CR) | 85.0 | 85.0 | 50.0 | 50.0 | - |
| Hypromellose type 2208 (Methocel^{™} K100M DC2) | - | - | - | - | 50.0 |
| Fumaric acid USP | 120.0 | - | - | 20.0 | 20.0 |
| Lactose monohydrate (Tablettose^{®} 80) | 85.0 | 85.0 | 50.0 | 50.0 | 50.0 |
| Microcrystalline cellulose (Avicel^{®} PH-102) | - | 60.0 | 33.75 | 23.75 | 23.75 |
| Calcium hydrogen phosphate dihydrate (Emcompress^{®} ) | - | 60.0 | 33.75 | 23.75 | 23.75 |
| Povidone (Plasdone^{®} K-29/32) | 15.0 | 15.0 | 15.0 | 15.0 | 15.0 |
| Silicon dioxide, colloidal (Aerosil^{®} 200) | 2.0 | 2.0 | 2.0 | 2.0 | 2.0 |
| Magnesium stearate (Ligamed^{®} MF-2) | 3.0 | 3.0 | 3.0 | 3.0 | 3.0 |
| Total tablet weight (mg) | 330.0 | 330.0 | 200.0 | 200.0 | 200.0 |

*By in vitro* dissolution testing, it was found that all tablet formulations exhibited extended-release of the active ingredient, with a duration of drug release extending over periods of more than 12 hours.

### Example 5: Matrix tablets comprising solid dispersions

The solid dispersion composition HME2 was prepared as described in Example 3 and incorporated in two matrix tablet formulations (5.1 and 5.2) whose compositions are provided in Table 4.

**Table 4**

| Matrix tablet formulation | 5.1 20 mg API | | 5.2 40 mg API | |
|---|---|---|---|---|
| Components (per unit) | [mg] | [wt.%] | [mg] | [wt.%] |
| HME2 | 100.00 | 27.78 | 200.00 | 31.25 |
| Hypromellose type 2208 (Methocel^{™} K100LV DC2) | 95.00 | 26.39 | - | - |
| Hypromellose type 2910 (Methocel^{™} E4M) | - | - | 150.00 | 23.44 |
| Hypromellose type 2208 (Methocel^{™} K100M DC2) | - | - | 40.00 | 6.25 |
| Microcrystalline cellulose (Avicel^{®} PH-102) | 80.00 | 22.22 | 120.00 | 18.75 |
| Calcium hydrogen phosphate dihydrate (Emcompress^{®}) | 80.00 | 22.22 | 120.00 | 18.75 |
| Silicon dioxide, colloidal (Aerosil^{®} 200) | 2.00 | 0.56 | 4.00 | 0.63 |
| Magnesium stearate (Ligamed^{®} MF-2) | 3.00 | 0.83 | 6.00 | 0.94 |
| Total | 360.00 | 100.00 | 640.00 | 100.00 |

As explained in the detailed description above, the hypromelloses in these formulations represent hydrophilic matrix-forming polymers. Microcrystalline cellulose and calcium hydrogen phosphate dihydrate function as tablet fillers, silicon dioxide colloidal primarily functions as glidant, and magnesium stearate primarily functions as lubricant.

Weighed amounts of the raw materials were mixed in several subsequent steps in a tumble blender (Turbula^{®} 2F): First, the milled extrudate, the fillers and the hydrophilic matrix-forming polymer(s) were mixed, then the glidant was added and mixed, and finally the lubricant was added and mixed. The resulting powder mixture was compressed on a lab-scale, single-punch pharmaceutical tablet press (Kilian Styl'One Evolution) into white to off-white, oval, biconvex tablets (17 mm x 7.2 mm) with brownish spots, using a main compression force in the range of 15 kN to 40 kN. The tablets had a mean thickness of about 5.7 mm, a mean resistance to crushing of about 110 N to 170 N and a friability of about 0.2 wt.% to 0.7 wt.%. An assay (HPLC-UV) indicated that the drug content was within the acceptable limits of 90% to 110% of the labelled amount. Total impurities were below 1%.

The drug release profiles of tablets of prototypes 5.1 and 5.2 were characterised by dissolution testing according to Ph. Eur. 2.9.3 / USP <711> using apparatus II (paddle apparatus). The dissolution medium was 1000 mL of USP phosphate buffer pH 6.8 with 0.3 % (w/v) sodium dodecyl sulphate. The paddle rotation speed was set at 100 rpm. Japanese sinkers were used for preventing the flotation of the tablets. Samples were withdrawn at intervals and analysed by HPLC with respect to their API concentration, from which the dissolved amounts of drug were calculated. The dissolution profiles are provided in Table 5.

**Table 5**

| Matrix tablet formulation | 5.1 20 mg API | 5.2 40 mg API |
|---|---|---|
| Sampling time [min] | API dissolved | API dissolved |
| 0 | 0.0 % | 0.0 % |
| 60 | 14.5 % | 18.0 % |
| 120 | 25.4 % | 24.2 % |
| 180 | 35.4 % | 30.3 % |
| 240 | 44.4 % | 36.7 % |
| 360 | 60.8 % | 50.4 % |
| 480 | 75.9 % | 63.9 % |
| 600 | 90.5 % | 76.1 % |
| 720 | 99.1 % | 89.1 % |
| 840 | 103.4 % | 93.9 % |
| 960 | 104.3 % | 98.4 % |
| 1080 | 103.9 % | 100.5 % |
| 1200 | 104.0 % | 102.3 % |
| 1320 | 104.0 % | 102.3 % |
| 1440 | 104.4 % | 103.1 % |

As the testing results illustrate, the matrix tablets incorporating a solid dispersion of the BK B2 receptor antagonist according to Formula (1) are capable of effecting extended release of the active ingredient over prolonged periods of time, such as over about 12 hours or even longer. Matrix tablet 5.1 exhibits a steeper dissolution profile than tablet 5.2 *in vitro.*

### Example 6: Human PK study

The basic pharmacokinetic profiles of the matrix tablet versions 5.1 and 5.2 after single administration were assessed and compared to immediate release capsules in a clinical phase I study, both under fasted and fed (HCHF-meal) conditions. The study was designed as a randomised, five period crossover study with 10 healthy volunteers. Table 6 lists key PK parameters found in the study.

**Table 6**

| Parameter | 5.1 fasted | 5.2 fasted | SMEDDS (fasted) | 5.1 fed | 5.2 fed |
|---|---|---|---|---|---|
| Cmax (mean) [ng/mL] | 89.8 | 111 | 272 | 128 | 160 |
| - SD of Cₘₐₓ [ng/mL] | 31.8 | 46.1 | 130 | 50.1 | 76.8 |
| - CV of Cₘₐₓ [%] | 35.4 | 41.5 | 47.8 | 39.1 | 48.1 |
| C₂₄ₕ (mean) [ng/mL] | 9.1 | 40.3 | 3.7 | 9.4 | 35.4 |
| - SD of C₂₄ₕ [ng/mL] | 6.9 | 25.5 | 4.2 | 8.3 | 36.1 |
| - CV of C₂₄ₕ (%) | 76.0 | 63.3 | 112.2 | 88.3 | 101.9 |
| tₘₐₓ (median) [h] | 4.0 | 9.0 | 1.0 | 6.0 | 11.0 |
| AUC₀₋₂₄ₕ (mean) [ng*h/mL] | 753 | 1427 | 896 | 941 | 1586 |
| - SD of AUC₀₋₂₄ₕ [ng*h/mL] | 314 | 644 | 510 | 565 | 795 |
| - CV of AUC₀₋₂₄ₕ [%] | 41.7 | 45.1 | 56.9 | 55.2 | 50.2 |
| AUC_{inf} (mean) [ng*h/mL] | 867 | 1896 | 937 | 1028 | 1955 |
| - SD of AUC_{inf} [ng*h/mL] | 408 | 899 | 556 | 572 | 1209 |
| - CV of AUC_{inf} [%] | 47.0 | 47.4 | 59.3 | 55.6 | 61.9 |

The data demonstrate that the matrix tablet formulations exhibit extended-release characteristics *in vivo,* as indicated e.g. by the tmax values. Prototype 5.2, which releases the active ingredient over a longer period of time *in vitro* compared to prototype 5.1 (see Example 5), also leads to a later tmax *in vivo,* both under fasted and fed conditions. Quite remarkably, the matrix tablets exhibit an overall drug exposure as the immediate release formulation (cf. AUC values), from which it can be concluded that the active ingredient, in spite of its unfavourable physical properties, is reliably released and absorbed even from the more distal regions of the intestines.

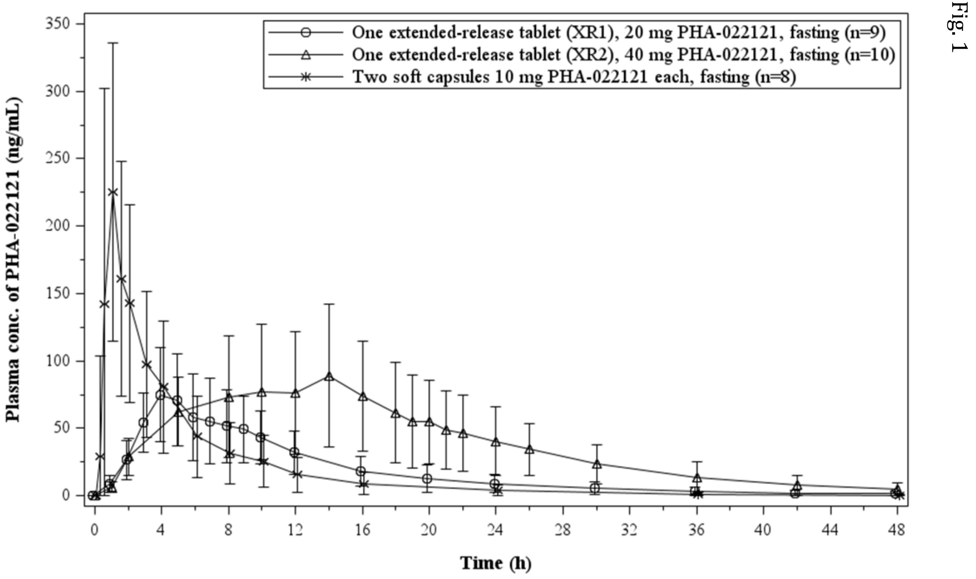

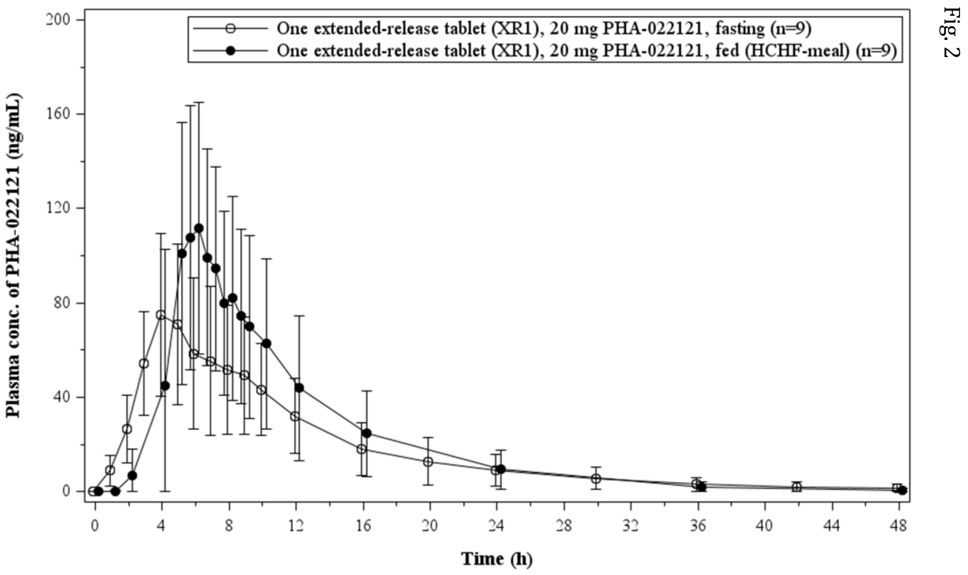

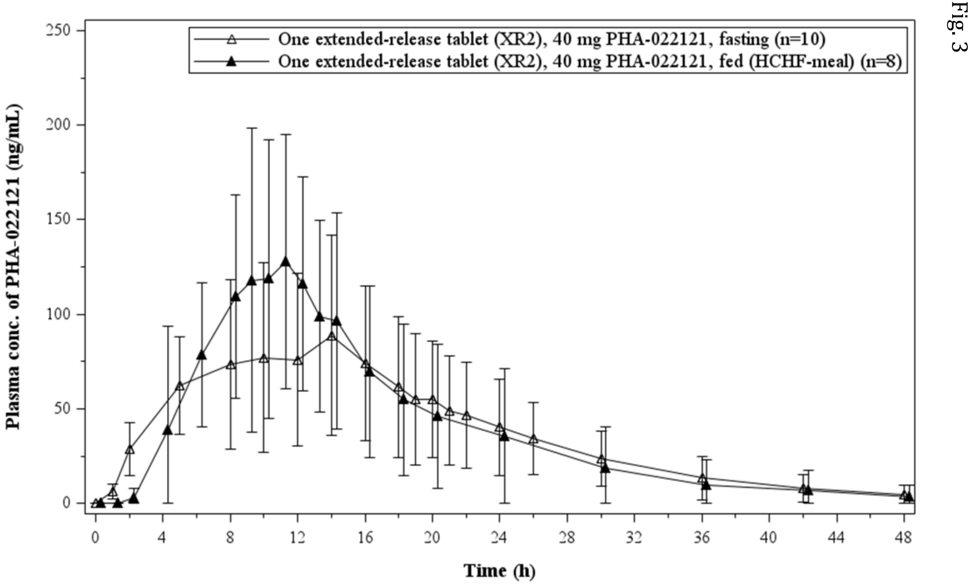

Figures 1 to 3 show the mean plasma concentration-over time profiles achieved by the respective formulations: Figure 1 depicts the plasma profiles after administration in the fasting state, whereas each of Figures 2 and 3 shows the plasma profiles after administration in the fed versus the fasting state for matrix tablet 5.1 (Figure 2) and matrix tablet 5.2 (Figure 3), respectively. Subsequent simulation studies confirmed the usefulness of the PK profiles of both tested matrix tablet formulations for continuous therapy using a once-daily or twice-daily dosing regimen. The SMEDDS formulation refers to immediate-release soft gelatin capsules comprising the active ingredient formulated as a self-microemulsifying drug delivery system (SMEDDS), by which a dose of 20 mg of the API was administered, as a comparator.

### Example 7: Film-coated matrix tablets

Tablets of prototypes 5.1 and 5.2 (see Example 5) were film-coated using an aqueous dispersion of a premix comprising the following constituents, the percentages being based on the total weight of the film constituents: Hydroxypropyl cellulose (7.00 wt.%), hypromellose (60.00 wt.%), MCC (15 wt.%), iron oxide yellow (10.00 wt.%), and glycerol (8.00 wt.%). The coating process was carried out in a perforated pan coater (Manesty XL 01 Lab) at an inlet temperature of 50-55 °C and an outlet temperature of 40-45 °C, using an atomising air pressure of 1000 mbar, a spray rate of 10-30 g/min and an inlet air volume of 350 m³/h. The spraying was conducted until the weight of the tablet cores had increased by 5 wt.%. In further batches using the same procedure, the tablet coatings contained microcrystalline cellulose (MCC) at level of 20 wt.% and iron oxide yellow at a level of 5 wt.%. The coated tablets had essentially the same extended-release characteristics as the uncoated tablet cores, apart from minor lag times caused by the coatings which are however irrelevant for the overall extended-release profile.

### Example 8: Matrix tablets comprising solid dispersions

Further batches of the solid dispersion composition HME2 were prepared as described in Example 3, except that in some batches, a screen size of 200 µm was used for the final phase of milling the pellets, which resulted in an average particle size D50 in the range of about 100 µm; and that for some tablets, the extrudates were milled by jet-milling (rather than using a hammer mill) to average particle sizes D50 in the range of about 50 µm and about 10 µm, respectively. The milled extrudates were incorporated in various matrix tablet formulations having dose strengths ranging from 1 mg to 50 mg. The matrix tablet compositions are provided in Tables 7 to 9.

In this context, an average particle size D50 in the range of about 200 µm, or ~200 µm, should be understood such that the respective particle size distribution, as determined gravimetrically by sieve analysis, has a median value between 125 µm and 250 µm. In other words, more than 50 wt.% of the milled particles pass a screen with an aperture width of 250 µm and less than 50 wt.% of the particles pass a screen width of 125 µm. Similarly, an average particle size D50 in the range of about 100 µm, or ~100 µm, should be understood as referring to a particle size distribution, as determined gravimetrically by sieve analysis, has a median value between 63 µm and 125 µm, in that more than 50 wt.% of the milled particles pass a screen with an aperture width of 125 µm and less than 50 wt.% of the particles pass a screen width of 63 µm. An average particle size D50 in the range of about 50 µm, or ~50 µm, should be understood as a D50 average in the range of about 50±25 µm, as determined by laser diffraction (such as with a particle size analyser LS 13 320 XR by Beckman Coulter), and an average particle size D50 in the range of about 10 µm, or ~10 µm, should be understood as a D50 average in the range of about 10±5 µm, also as determined by laser diffraction.

These matrix tablet formulations provide, *inter alia,* further examples of extended-release matrices incorporating a compound of Formula (1) formed by a single grade of a hypromellose polymer, in particular a high viscosity grade of a hypromellose type 2208 polymer (such as Methocel^{™} K100M DC2 or Methocel^{™} K 100M Premium; or by a combination of hypromellose polymers, such as a combination of a hypromellose type 2208 and a hypromellose type 2910; for example a high viscosity grade of a hypromellose type 2208 in combination with either a high viscosity grade or a low viscosity grade of a hypromellose type 2910 (such as Methocel^{™} E4M).

The matrix tablet formulations further provide examples of additional excipients that could be used to modify the physical properties of the tablet, such as macrogols, special low viscosity grades of hyprolose or hypromellose, or acid-insoluble polymers such as HPMCAS. Moreover, the examples show how a tablet filler (or filler-binder) such as MCC and a glidant such as colloidal silica can together be replaced by a multifunctional excipient, in this case silicified MCC.

**Table 7**

| Tablet formulation | 7.1 | 7.2 | 7.3 | 7.4 | 7.5 |
|---|---|---|---|---|---|
| API dose strength [mg] | 40 | 40 | 40 | 40 | 40 |
| Tablet weight [mg] | 640 | 640 | 640 | 640 | 640 |
| Tablet dimensions [mm] | 16.6 x 7.2 | 16.6 x 7.2 | 16.6 x 7.2 | 16.6 x 7.2 | 16.6 x 7.2 |
| D50 of HME2 [µm] | ~200 | ~100 | ~100 | ~100 | ~100 |

| Components [wt.%] | | | | | |
|---|---|---|---|---|---|
| HME2 | 31.25 | 31.25 | 31.25 | 31.25 | 31.25 |
| Hypromellose type 2910 (Methocel^{™} E4M) | 23.44 | 23.44 | 23.44 | 23.44 | 23.44 |
| Hypromellose type 2208 (Methocel^{™} K100M Premium) | 6.25 | 6.25 | 6.25 | 6.25 | 6.25 |
| Hypromellose type 2910 (BonuCel^{®} D 5 H) | 5.00 | | | | 5.00 |
| Hyprolose (HPC-SSL) | | 5.00 | | | |
| Macrogol 6000 | | | 5.00 | | |
| HPMCAS (Aqoat-AS-MMP) | | | | 5.00 | |
| MCC (Avicel^{®} PH-102) | 14.22 | 14.22 | 14.22 | 14.22 | |
| MCC (Vivapu^{®}r 105) | 6.25 | 6.25 | 6.25 | 6.25 | 20.47 |
| Silicified MCC (Prosolv^{®} SMCC 50) | | | | | |
| Calcium hydrogen phosphate dihydrate (Emcompress^{®}) | 12.66 | 12.66 | 12.66 | 12.66 | 12.66 |
| Silicon dioxide, colloidal (Aerosil^{®} 200) | 0.63 | 0.63 | 0.63 | 0.63 | 0.63 |
| Magnesium stearate (Ligamed^{®} MF-2) | 0.31 | 0.31 | 0.31 | 0.31 | 0.31 |
| Total | 100.00 | 100.00 | 100.00 | 100.00 | 100.00 |

**Table 8**

| Tablet formulation | 7.6 | 7.7 | 7.8 | 7.9 | 7.10 |
|---|---|---|---|---|---|
| API dose strength [mg] | 40 | 40 | 40 | 20 | 20 |
| Tablet weight [mg] | 640 | 640 | 640 | 540 | 320 |
| Tablet dimensions [mm] | 16.6 x 7.2 | 16.6 x 7.2 | 16.6 x 7.2 | 16.6 x 7.2 | 13.5 x 6.5 |
| D50 of HME2 [µm] | ~100 | ~50 | ~10 | ~200 | ~200 |

| Components [wt.%] | | | | | |
|---|---|---|---|---|---|
| HME2 | 31.25 | 31.25 | 31.25 | 18.52 | 31.25 |
| Hypromellose type 2910 (Methocel^{™} E4M) | 23.44 | 23.44 | 23.44 | 27.78 | 23.44 |
| Hypromellose type 2208 (Methocel^{™} K100M Premium) | 6.25 | 6.25 | 6.25 | 7.41 | 6.25 |
| Hypromellose type 2910 (BonuCel^{®} D 5 H) | 5.00 | 5.00 | 5.00 | | |
| Hyprolose (HPC-SSL) | | | | | |
| Macrogol 6000 | | | | | |
| HPMCAS (Aqoat^{®}-AS-MMP) | | | | | |
| MCC (Avicel^{®} PH-102) | | 14.22 | 14.22 | 22.41 | 18.91 |
| MCC (Vivapur^{®} 105) | | 6.25 | 6.25 | | |
| Silicified MCC (Prosolv^{®} SMCC 50) | 20.78 | | | | |
| Calcium hydrogen phosphate dihydrate (Emcompress^{®}) | 12.66 | 12.66 | 12.66 | 22.41 | 18.91 |
| Silicon dioxide, colloidal (Aerosil^{®} 200) | 0.31 | 0.63 | 0.63 | 0.74 | 0.63 |
| Magnesium stearate (Ligamed^{®} MF-2) | 0.31 | 0.31 | 0.31 | 0.74 | 0.63 |
| Total | 100.00 | 100.00 | 100.00 | 100.00 | 100.00 |

**Table 9**

| Tablet formulation | 7.11 | 7.12 | 7.13 | 7.14 | 7.15 | 7.16 |
|---|---|---|---|---|---|---|
| API dose strength [mg] | 20 | 20 | 30 | 50 | 1 | 20 |
| Tablet weight [mg] | 320 | 320 | 590 | 690 | 25 | 425 |
| Tablet dimensions [mm] | 13.5 x 6.5 | 13.5 x 6.5 | 16.6 x 7.2 | 16.6 x 7.2 | 3.0* | 14 x 7 |
| D50 of HME2 [µm] | ~200 | ~200 | ~200 | ~200 | ~100 | ~100 |

| Components [wt.%] | | | | | | |
|---|---|---|---|---|---|---|
| HME2 | 31.25 | 31.25 | 25.42 | 36.23 | 20.00 | 23.53 |
| Hypromellose type 2910 (Methocel^{™} E4M) | 6.25 | | 25.42 | 21.74 | | |
| Hypromellose type 2208 (Methocel^{™} K100M Premium) | 23.44 | 29.69 | 6.78 | 5.80 | 32.00 | 35.29 |
| Hypromellose type 2910 (BonuCel^{®} D 5 H) | | | | | | |
| Hyprolose (HPC-SSL) | | | | | | |
| Macrogol 6000 | | | | | | |
| HPMCAS (Agoat^{®} -AS-MMP) | | | | | | |
| MCC (Avicel^{®} PH-102) | 18.91 | 18.91 | 20.51 | 17.54 | | 20.00 |
| MCC (Vivapur^{®} 105) | | | | | 22.00 | |
| Silicified MCC (Prosolv^{®} SMCC 50) | | | | | | |
| Calcium hydrogen phosphate dihydrate (Emcompress^{®}) | 18.91 | 18.91 | 20.51 | 17.54 | 22.00 | 20.00 |
| Silicon dioxide, colloidal (Aerosil^{®} 200) | 0.63 | 0.63 | 0.68 | 0.58 | 1.00 | 0.71 |
| Magnesium stearate (Ligamed^{®} MF-2) | 0.63 | 0.63 | 0.68 | 0.58 | 1.00 | 0.47 |
| Talc | | | | | 2.00 | |
| Total | 100.00 | 100.00 | 100.00 | 100.00 | 100.00 | 100.00 |

| | | | | | | |
|---|---|---|---|---|---|---|
| *Diameter of round tablet | | | | | | |

The *in-vitro* drug release profiles of several prototype formulations were tested using a dissolution test device (also referred to as "stress tester" or "dissolution stress tester") as described in G. Garbacz et al., Expert Opin. Drug Deliv. vol. 7, 2010, pages 1251-1261. In short, the device comprises a central apparatus with dissolution vessels in which spherical probe chambers made of stainless-steel wire netting holding the test specimen are accommodated. Each chamber has a bottom part which is screwed into the central pipe-like axle by a bush and a nozzle. In the working mode, the central pipe is placed on the deck plate of the device about 3 mm above the top edges of a series of standard dissolution vessels in their symmetry plane. Each probe chamber operates in a separate vessel. At one end, the central axis itself is coupled to a pressure regulation device, and at the other end, a stepping motor is attached. Pressure waves are generated by periodic inflation and deflation of balloons located inside the probe chambers. These are controlled by synchronized switching of valves and the pressure can be regulated by a pressure-reducing device. The central axis is driven by the stepping motor, which can be programmed to position the probe chambers inside and outside of the dissolution vessel, resulting in immersion in the medium or in contact with air. The dissolution medium (typically 1160 mL) is mixed by a separate paddle stirrer (typically operated at 200 rpm).

Two different stress dissolution testing programmes using this device were conducted, which are briefly outlined as "Method 1" and "Method 2" below. In both protocols, Simulated Gastric Fluid (SGF) pH 1.8 without pepsin according to USP was used as dissolution medium for the gastric phases, and Modified Fasted State Simulated Intestinal Fluid (mFaSSIF) pH 6.8 was used as dissolution medium for the intestinal transit phases.

Method 1, which is described in Table 10, is intended to simulate drug administration in the fasted state with relatively low gastrointestinal motility conditions, whereas Method 2 simulates administration in the fasted state with high intestinal motility or enhanced stress conditions. Method 2 differs from Method 1 in that the colonic phase comprises an additional agitation and stress event (two pressure waves of 200 mbar, 30 s rotation at 50 rpm) at 7 h. Moreover, additional agitation events (30 s rotation at 50 rpm) occurred at 0.75, 1.25, 1.75, 2.25, 2.75, 3.25, 3.75, 4.25, 4.75, 5.25, 5.75, 6.25, 6.75, 7.25, 7.75, 8.25, 8.75, 9.25, 9.75, 10.25, 10.75, 11.25, 11.75, 12.5, 13.5, 14.5, 15.5, 16.5, 17.5, 18.5, 19.5, 20.5, 21.5, 22.5 and 23.5 h, and additional minor stress events (one pressure wave of 150 mbar plus 30 s rotation at 50 rpm) occurred at 1.5, 2.5, 3.5, 4.5, 5.5, 6.5, 7.5, 8.5, 9.5, 10.5, 11.5, 13, 14, 15, 17, 18, 19, 21, 22 and 23 h.

Samples of the dissolution medium were taken every 60 min up to 24 h into HPLC vials via PE filters (1 µm pore size). Subsequently, the drug substance concentrations were determined by HPLC.

**Table 10**

| Phase | Time | Medium | Agitation/Stress |
|---|---|---|---|
| Gastric | 0.0-0.5 h | SGF pH 1.8 | None |
| Gastric emptying | at 0.5 h | SGF pH 1.8 | Three pressure waves of 300 mbar and 60 s rotation at 100 rpm |
| Small intestinal | 0.5-5.0 h | mFaSSIF pH 6.8 fully exchanged at 4 h | At 1, 2, and 3 h: |
| | | | One pressure wave of 150 mbar and 30 s rotation at 50 rpm |
| Ileocaecal transit | at 5.0 h | mFaSSIF pH 6.8 | Two pressure waves of 200 mbar and 30 s rotation at 50 rpm |
| Colonic | 5.0-24.0 h | mFaSSIF pH 6.8 fully exchanged at 8 and 12 h | At 9, 12, 16, 20, and 24 h: |
| | | | Two pressure waves of 200 mbar and 30 s rotation at 50 rpm |

Testing results are presented in Tables 11 and 12. The results provided in Table 11 were obtained with Method 1, whereas the results of Table 12 were obtained with Method 2.

**Table 11**

| Tablet | 7.9 | 7.10 | 7.11 | 7.12 | 7.13 | 7.14 |
|---|---|---|---|---|---|---|
| t [h] | [%] | [%] | [%] | [%] | [%] | [%] |
| 0 | 0.0 | 0.0 | 0.0 | 0.0 | 0.0 | 0.0 |
| 1 | 0.8 | 0.6 | 1.2 | 1.2 | 2.9 | 2.5 |
| 2 | 3.7 | 3.5 | 3.5 | 4.4 | 7.5 | 5.7 |
| 3 | 7.5 | 7.7 | 7.2 | 8.4 | 12.0 | 10.0 |
| 6 | 20.2 | 29.8 | 23.3 | 24.8 | 31.9 | 25.9 |
| 9 | 28.0 | 40.0 | 31.8 | 32.9 | 40.2 | 33.3 |
| 12 | 40.1 | 58.9 | 49.2 | 48.8 | 49.5 | 43.3 |
| 18 | 69.4 | 94.8 | 82.8 | 80.8 | 82.9 | 68.2 |
| 24 | 92.4 | 99.9 | 101.5 | 99.1 | 109.0 | 82.2 |

Note: Each value represents the mean of three tested tablets.

**Table 12**

| Tablet | 7.1a Cores | 7.1a Coated | 7.1b Cores | 7.1b Coated | 7.1c Cores | 7.1c Coated |
|---|---|---|---|---|---|---|
| t [h] | [%] | [%] | [%] | [%] | [%] | [%] |
| 0 | 0.0 | 0.0 | 0.0 | 0.0 | 0.0 | 0.0 |
| 1 | 0.4 | 0.4 | 1.1 | 1.1 | 1.7 | 1.2 |
| 2 | 2.8 | 2.4 | 5.2 | 5.1 | 5.5 | 5.1 |
| 3 | 6.8 | 5.9 | 10.6 | 10.3 | 9.8 | 10.4 |
| 6 | 24.3 | 23.4 | 29.0 | 28.6 | 25.7 | 25.9 |
| 9 | 38.5 | 36.8 | 42.0 | 42.3 | 38.2 | 39.0 |
| 12 | 53.9 | 52.3 | 58.9 | 59.4 | 52.2 | 54.6 |
| 18 | 76.5 | 76.4 | 79.8 | 80.0 | 75.0 | 77.2 |
| 24 | 95.3 | 95.8 | 94.3 | 94.5 | 93.9 | 95.8 |

Notes: Each value represents the mean of four or five tested tablets. 7.1a, 7.1b and 7.1c are different batches of the same matrix tablet formulation 7.1.

The results clearly indicate that the tested matrix tablets are all suitable as extended-release formulations for the oral delivery of this active ingredient where the desired release occurs over a long period, i.e. over 12 hours, or even up to 24 hours. The comparative results shown in Table 12 further indicate that the design and composition of the matrix governs the release rate, and that the coating only has a negligible effect on the overall release profile.

## Claims

1. A pharmaceutical matrix tablet comprising a bradykinin (BK) B2 receptor antagonist having a chemical structure according to Formula (1), or a stereoisomer, salt or solvate thereof: 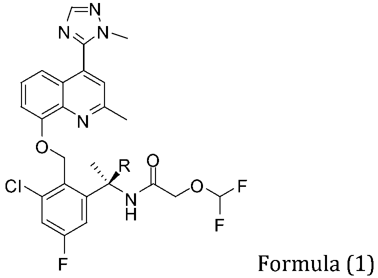 wherein R is deuterium; and at least one hydrophilic matrix-forming polymer selected from hydroxypropyl cellulose, hydroxypropyl methylcellulose, hydroxypropyl methylcellulose acetate succinate, alginic acid, bentonite, carbomer, carboxymethylcellulose calcium, carboxymethyl cellulose sodium, carrageenan, cellulose, copovidone, gelatin, guar gum, hydroxyethylcellulose, hydroxypropyl starch, methylcellulose, pectin, polyethylene oxide, polymethacrylates, potassium (or calcium, or sodium) alginate, povidone, tragacanth, or xanthan gum.

2. The pharmaceutical matrix tablet of claim 1, consisting of an optionally coated single-layer tablet.

3. The pharmaceutical matrix tablet of claim 1 or 2, wherein the matrix tablet is an extended-release matrix tablet.

4. The pharmaceutical matrix tablet of any one of the preceding claims, wherein the hydrophilic matrix-forming polymer is. selected from hydroxypropyl cellulose, hydroxypropyl methylcellulose, and combinations thereof; such as wherein the hydrophilic matrix-forming polymer comprises, or consists of, a combination of a hydroxypropyl methylcellulose type 2208 and a hydroxypropyl methylcellulose type 2910.

5. The pharmaceutical matrix tablet of claim 4, comprising 20 wt.% to 40 wt.% of hydroxypropyl methylcellulose, or of a combination of different grades of hydroxypropyl methylcellulose.

6. The pharmaceutical matrix tablet of any one of the preceding claims, further comprising a tablet filler selected from microcrystalline cellulose and/or calcium hydrogen phosphate dihydrate; preferably wherein the microcrystalline cellulose is silicified.

7. The pharmaceutical matrix tablet of any one of the preceding claims, wherein the BK B2 receptor antagonist is incorporated:
(a) in micronised form; or
(b) in solubilised form; such as wherein the BK B2 receptor antagonist is incorporated predominantly in the amorphous form.

8. The pharmaceutical matrix tablet according to item (b) of claim 7, comprising a solid dispersion of the amorphous BK B2 receptor antagonist in a carrier, wherein the carrier comprises at least one pharmaceutically acceptable carrier polymer selected from hydroxypropyl methylcellulose, hydroxypropyl methylcellulose acetate succinate, copovidone and combinations thereof.

9. The pharmaceutical matrix tablet of claim8, wherein the solid dispersion is incorporated as particles having an average particle size D50 of not more than 250 µm; and/or
wherein the solid dispersion comprises at least 10 wt.% of BK B2 receptor antagonist, relative to the total weight of the solid dispersion; and/or
wherein the matrix tablet comprises 15 wt.% to 40 wt.% of the solid dispersion, relative to the total weight of the tablet without its optional coating; and/or
wherein the weight ratio of the solid dispersion to the hydrophilic matrix-forming polymer is in the range from 2 : 1 to 1 : 2.

10. The pharmaceutical matrix tablet of claim 8 or 9, comprising:
- 15 wt.% to 40 wt.% of the solid dispersion,
- 20 wt.% to 40 wt.% of the hydrophilic matrix-forming polymer, and
- 30 wt.% to 50 wt.% of the filler; such ascomprising:
- 20 wt.% to 40 wt.% of the solid dispersion,
- 20 wt.% to 40 wt.% of hydroxypropyl methylcellulose, or of a combination of different grades of hydroxypropyl methylcellulose,
- 30 wt.% to 50 wt.% of microcrystalline cellulose, calcium hydrogen phosphate dihydrate, or a combination thereof,
- up to 2 wt.% of a glidant, in particular colloidal silicon dioxide, and
- up to 2 wt.% of a lubricant, in particular magnesium stearate.

11. The pharmaceutical matrix tablet of any one of the preceding claims, comprising a coating, said coating comprising a water-soluble film-forming polymer and a plasticiser; such as wherein the amount of the coating is 2 wt.% to 8 wt.% relative to the weight of the uncoated tablet.

12. A method for preparing the matrix tablet of any one of the preceding claims, comprising the steps of:
(a) providing a solid dispersion in powder or granular form, the solid dispersion comprising BK B2 receptor antagonist and a carrier comprising at least one pharmaceutically acceptable carrier polymer, wherein the BK B2 receptor antagonist is amorphous and dispersed in the carrier;
(b) combining the solid dispersion with a hydrophilic matrix-forming polymer, a filler and optionally further excipients such as to form a compressible powder or granule mixture, and
(c) compressing the powder or granule mixture to obtain the matrix tablet; preferably wherein said compressing is conducted without any prior agglomeration step.

13. The pharmaceutical matrix tablet of any one claims 1 to 11 for use in the treatment of a subject suffering from a disease or condition responsive to BK B2 receptor modulation, wherein the disease or condition responsive to BK B2 receptor modulation is selected from the group comprising a skin disorder; eye disease; ear disease; mouth, throat and respiratory disease; gastrointestinal disease; liver, gallbladder and pancreatic disease; urinary tract and kidney disease; disease of male genital organs and female genital organs; disease of the hormone system; metabolic disease; cardiovascular disease; blood disease; lymphatic disease; disorder of the central nervous system; brain disorder; musculoskeletal system disease; allergy disorder; pain; infectious disease; inflammatory disorder; injury; immunology disorder; cancer; hereditary disease; edema; and capillary leak syndrome.

14. The pharmaceutical matrix tablet for use according to claim 13, wherein the disease or condition responsive to BK B2 receptor modulation is angioedema, such as hereditary angioedema (HAE), preferably type I HAE, type II HAE or type III HAE, more preferably type I HAE or type II HAE, acquired angioedema (AAE), non-histaminergic idiopathic angioedema, allergic angioedema, drug-induced angioedema or angioedema of unidentified cause.

15. The pharmaceutical matrix tablet for use according to claim 13 or 14, wherein the treatment is chronic treatment over at least one month.
